# EUROPEAN PATENT SPECIFICATION

(11) **EP 1 648 506 B1**
(45) Date of publication and mention of the grant of the patent: **15.02.2012**
(21) Application number: 04713982.9
(22) Date of filing: 24.02.2004
(51) Int. Cl.: C07K 16/28, C07K 16/30, C12Q 1/68, A61K 39/395, A61P 35/00, G01N 33/574, G01N 33/577

(54) **METHOD FOR TREATING SYNOVIAL SARCOMA**
VERFAHREN ZUR BEHANDLUNG VON SYNOVIALSARKOM
METHODE DE TRAITEMENT DU SYNOVIALOME

(30) Priority: 11.07.2003 US 486195 P
(43) Date of publication of application: 26.04.2006
(73) Proprietor: Oncotherapy Science, Inc., Kawasaki-shi Kanagawa 213-0012 (JP)
(72) Inventor: NAKAMURA, Yusuke, Tokyo 146-0085 (JP); KATAGIRI, Toyomasa c/o Institute for Genome Research, Tokushima 770-8503 (JP); KUHARA, Motoki, Aichi 460-0008 (JP)
(74) Representative: Woods, Geoffrey Corlett
(86) International application number: PCT/JP2004/002144
(87) International publication number: WO 2005/004912

(56) References cited:
- WO-A-02/055705
- WO-A-02/092635
- WO-A2-96/02641
- J. KOIKE ET AL.: "Molecular cloning of Frizzled-10, a novel member of the Frizzled gene family." BIOCHEMICAL AND BIOPHYSICAL RESEARCH COMMUNICATIONS, vol. 262, no. 1, 19 August 1999 (1999-08-19), pages 39-43, XP002934756 DULUTH, MN, USA

### SEQUENCE LISTING

<110> ONCOTHERAPY SCIENCE, INC.
   JAPAN AS REPRESENTED BY PRESIDENT OF THE UNIVERSITY 0F TOKYO
<120> METHOD FOR TREATING SYNOVIAL SARCOMA
<130> PH-1999-PCT
<140> US 60/486,195
   <141> 2003-07-11
<160> 24
<170> PatentIn version 3.1
<210> 1
   <211> 581
   <212> PRT
   <213> Homo sapiens
<400> 1
   Met Gln Arg Pro Gly Pro Arg Leu Trp Leu Val Leu Gln Val Met Gly
   1               5                   10                  15
   Ser Cys Ala Ala Ile Ser Ser Met Asp Met Glu Arg Pro Gly Asp Gly
               20                  25                  30
   Lys Cys Gln Pro Ile Glu Ile Pro Met Cys Lys Asp Ile Gly Tyr Asn
           35                  40                  45
   Met Thr Arg Met Pro Asn Leu Met Gly His Glu Asn Gln Arg Glu Ala
       50                  55                  60
   Ala Ile Gln Leu His Glu Phe Ala Pro Leu Val Glu Tyr Gly Cys His
   65                  70                  75                  80
   Gly His Leu Arg Phe Phe Leu Cys Ser Leu Tyr Ala Pro Met Cys Thr
   85 90 95
   Glu Gln Val Ser Thr Pro Ile Pro Ala Cys Arg Val Met Cys Glu Gln
   100 105 110
   Ala Arg Leu Lys Cys Ser Pro Ile Met Glu Gln Phe Asn Phe Lys Trp
   115 120 125
   Pro Asp Ser Leu Asp Cys Arg Lys Leu Pro Asn Lys Asn Asp Pro Asn
   130 135 140
   Tyr Leu Cys Met Glu Ala Pro Asn Asn Gly Ser Asp Glu Pro Thr Arg
   145 150 155 160
   Gly Ser Gly Leu Phe Pro Pro Leu Phe Arg Pro Gln Arg Pro His Ser
   165 170 175
   Ala Gln Glu His Pro Leu Lys Asp Gly Gly Pro Gly Arg Gly Gly Cys
   180 185 190
   Asp Asn Pro Gly Lys Phe His His Val Glu Lys Ser Ala Ser Cys Ala
   195 200 205
   Pro Leu Cys Thr Pro Gly Val Asp Val Tyr Trp Ser Arg Glu Asp Lys
   210 215 220
   Arg Phe Ala Val Val Trp Leu Ala Ile Trp Ala Val Leu Cys Phe Phe
   225 230 235 240
   Ser Ser Ala Phe Thr Val Leu Thr Phe Leu Ile Asp Pro Ala Arg Phe
   245 250 255
   Arg Tyr Pro Glu Arg Pro Ile Ile Phe Leu Ser Met Cys Tyr Cys Val
   260 265 270
   Tyr Ser Val Gly Tyr Leu Ile Arg Leu Phe Ala Gly Ala Glu Ser Ile
   275 280 285
   Ala Cys Asp Arg Asp Ser Gly Gln Leu Tyr Val Ile Gln Glu Gly Leu
   290 295 300
   Glu Ser Thr Gly Cys Thr Leu Val Phe Leu Val Leu Tyr Tyr Phe Gly
   305 310 315 320
   Met Ala Ser Ser Leu Trp Trp Val Val Leu Thr Leu Thr Trp Phe Leu
   325 330 335
   Ala Ala Gly Lys Lys Trp Gly His Glu Ala Ile Glu Ala Asn Ser Ser
   340 345 350
   Tyr Phe His Leu Ala Ala Trp Ala Ile Pro Ala Val Lys Thr Ile Leu
   355 360 365
   Ile Leu Val Met Arg Arg Val Ala Gly Asp Glu Leu Thr Gly Val Cys
   370 375 380
   Tyr Val Gly Ser Met Asp Val Asn Ala Leu Thr Gly Phe Val Leu Ile
   385 390 395 400
   Pro Leu Ala Cys Tyr Leu Val Ile Gly Thr Ser Phe Ile Leu Ser Gly
   405 410 415
   Phe Val Ala Leu Phe His Ile Arg Arg Val Met Lys Thr Gly Gly Glu
   420 425 430
   Asn Thr Asp Lys Leu Glu Lys Leu Met Val Arg Ile Gly Leu Phe Ser
           435                     440                     445
   Val Leu Tyr Thr Val Pro Ala Thr Cys Val Ile Ala Cys Tyr Phe Tyr
       450                     455                     460
   Glu Arg Leu Asn Met Asp Tyr Trp Lys Ile Leu Ala Ala Gln His Lys
   465                     470                     475                 480
   Cys Lys Met Asn Asn Gln Thr Lys Thr Leu Asp Cys Leu Met Ala Ala
                   485                     490                     495
   Ser Ile Pro Ala Val Glu Ile Phe Met Val Lys Ile Phe Met Leu Leu
               500                     505                     510
   Val Val Gly Ile Thr Ser Gly Met Trp Ile Trp Thr Ser Lys Thr Leu
           515                     520                     525
   Gln Ser Trp Gln Gln Val Cys Ser Arg Arg Leu Lys Lys Lys Ser Arg
       530                     535                     540
   Arg Lys Pro Ala Ser Val Ile Thr Ser Gly Gly Ile Tyr Lys Lys Ala
   545                     550                     555                 560
   Gln His Pro Gln Lys Thr His His Gly Lys Tyr Glu Ile Pro Ala Gln
                   565                     570                     575
   Ser Pro Thr Cys Val
               580
<210> 2
   <211> 2811
   <212> DNA
   <213> Homo sapiens
<400> 2
   acacgtccaa cgccagcatg cagcgcccgg gcccccgcct gtggctggtc ctgcaggtga      60
   tgggctcgtg cgccgccatc agctccatgg acatggagcg cccgggcgac ggcaaatgcc     120
   agcccatcga gatcccgatg tgcaaggaca tcggctacaa catgactcgt atgcccaacc     180
   tgatgggcca cgagaaccag cgcgaggcag ccatccagtt gcacgagttc gcgccgctgg     240
   tggagtacgg ctgccacggc cacctccgct tcttcctgtg ctcgctgtac gcgccgatgt     300
   gcaccgagca ggtctctacc cccatccccg cctgccgggt catgtgcgag caggcccggc     360
   tcaagtgctc cccgattatg gagcagttca acttcaagtg gcccgactcc ctggactgcc     420
   ggaaactccc caacaagaac gaccccaact acctgtgcat ggaggcgccc aacaacggct     480
   cggacgagcc cacccggggc tcgggcctgt tcccgccgct gttccggccg cagcggcccc     540
   acagcgcgca ggagcacccg ctgaaggacg ggggccccgg gcgcggcggc tgcgacaacc     600
   cgggcaagtt ccaccacgtg gagaagagcg cgtcgtgcgc gccgctctgc acgcccggcg     660
   tggacgtgta ctggagccgc gaggacaagc gcttcgcagt ggtctggctg gccatctggg     720
   cggtgctgtg cttcttctcc agcgccttca ccgtgctcac cttcctcatc gacccggccc     780
   gcttccgcta ccccgagcgc cccatcatct tcctctccat gtgctactgc gtctactccg     840
   tgggctacct catccgcctc ttcgccggcg ccgagagcat cgcctgcgac cgggacagcg     900
   gccagctcta tgtcatccag gagggactgg agagcaccgg ctgcacgctg gtcttcctgg     960
   tcctctacta cttcggcatg gccagctcgc tgtggtgggt ggtcctcacg ctcacctggt    1020
   tcctggccgc cggcaagaag tggggccacg aggccatcga agccaacagc agctacttcc    1080
   acctggcagc ctgggccatc ccggcggtga agaccatcct gatcctggtc atgcgcaggg    1140
   tggcggggga cgagctcacc ggggtctgct acgtgggcag catggacgtc aacgcgctca 1200
   ccggcttcgt gctcattccc ctggcctgct acctggtcat cggcacgtcc ttcatcctct 1260
   cgggcttcgt ggccctgttc cacatccgga gggtgatgaa gacgggcggc gagaacacgg 1320
   acaagctgga gaagctcatg gtgcgtatcg ggctcttctc tgtgctgtac accgtgccgg 1380
   ccacctgtgt gatcgcctgc tacttttacg aacgcctcaa catggattac tggaagatcc 1440
   tggcggcgca gcacaagtgc aaaatgaaca accagactaa aacgctggac tgcctgatgg 1500
   ccgcctccat ccccgccgtg gagatcttca tggtgaagat ctttatgctg ctggtggtgg 1560
   ggatcaccag cgggatgtgg atttggacct ccaagactct gcagtcctgg cagcaggtgt 1620
   gcagccgtag gttaaagaag aagagccgga gaaaaccggc cagcgtgatc accagcggtg 1680
   ggatttacaa aaaagcccag catccccaga aaactcacca cgggaaatat gagatccctg 1740
   cccagtcgcc cacctgcgtg tgaacagggc tggagggaag ggcacagggg cgcccggagc 1800
   taagatgtgg tgcttttctt ggttgtgttt ttctttcttc ttcttctttt tttttttttt 1860
   ataaaagcaa aagagaaata cataaaaaag tgtttaccct gaaattcagg atgctgtgat 1920
   acactgaaag gaaaaatgta cttaaagggt tttgttttgt tttggttttc cagcgaaggg 1980
   aagctcctcc agtgaagtag cctcttgtgt aactaatttg tggtaaagta gttgattcag 2040
   ccctcagaag aaaacttttg tttagagccc tccgtaaata tacatctgtg tatttgagtt 2100
   ggctttgcta cccatttaca aataagagga cagataactg ctttgcaaat tcaagagcct 2160
   cccctgggtt aacaaatgag ccatccccag ggcccacccc caggaaggcc acagtgctgg 2220
   gcggcatccc tgcagaggaa agacaggacc cggggcccgc ctcacacccc agtggatttg 2280
   gagttgctta aaatagactc tggccttcac caatagtctc tctgcaagac agaaacctcc 2340
   atcaaacctc acatttgtga actcaaacga tgtgcaatac atttttttct ctttccttga 2400
   aaataaaaag agaaacaagt attttgctat atataaagac aacaaaagaa atctcctaac 2460
   aaaagaacta agaggcccag ccctcagaaa cccttcagtg ctacattttg tggcttttta 2520
   atggaaacca agccaatgtt atagacgttt ggactgattt gtggaaagga ggggggaaga 2580
   gggagaagga tcattcaaaa gttacccaaa gggcttattg actctttcta ttgttaaaca 2640
   aatgatttcc acaaacagat caggaagcac taggttggca gagacacttt gtctagtgta 2700
   ttctcttcac agtgccagga aagagtggtt tctgcgtgtg tatatttgta atatatgata 2760
   tttttcatgc tccactattt tattaaaaat aaaatatgtt ctttaaaaaa a 2811
<210> 3
   <211> 23
   <212> DNA
   <213> Artificial
<220>
   <223> synthetic oligonucleotide
<400> 3
   ctcgaggttt cctcactaga caa 23
<210> 4
   <211> 23
   <212> DNA
   <213> Artificial
<220>
   <223> synthetic oligonucleotide
<400> 4
   aatggttaaa ccgccctaaa taa 23
<210> 5
   <211> 21
   <212> DNA
   <213> Artificial
<220>
   <223> synthetic oligonucleotide
<400> 5
   tccaccttct tcactgtcac c 21
<210> 6
   <211> 23
   <212> DNA
   <213> Artificial
<220>
   <223> synthetic oligonucleotide
<400> 6
   taaaatacgg agtctgtagg ggc 23
<210> 7
   <211> 23
   <212> DNA
   <213> Artificial
<220>
   <223> synthetic oligonucleotide
<400> 7
   attgaatagg cctgatcatc tga 23
<210> 8
   <211> 23
   <212> DNA
   <213> Artificial
<220>
   <223> synthetic oligonucleotide
<400> 8
   ataggagcgt agagtgcaca aag 23
<210> 9
   <211> 21
   <212> DNA
   <213> Artificial
<220>
   <223> synthetic oligonucleotide
<400> 9
   atgacttaca gatcccccga c 21
<210> 10
   <211> 21
   <212> DNA
   <213> Artificial
<220>
   <223> synthetic oligonucleotide
<400> 10
   acagagcagg ggaagtcaca t 21
<210> 11
   <211> 22
   <212> DNA
   <213> Artificial
<220>
   <223> synthetic oligonucleotide
<400> 11
   ctgcgcttct tcctatgcac ta 22
<210> 12
   <211> 22
   <212> DNA
   <213> Artificial
<220>
   <223> synthetic oligonucleotide
<400> 12
   ttgttgtaga gcgggtgtga ct 22
<210> 13
   <211> 23
   <212> DNA
   <213> Artificial
<220>
   <223> synthetic oligonucleotide
<400> 13
   cgctactttg tactcttgcc act 23
<210> 14
   <211> 23
   <212> DNA
   <213> Artificial
<220>
   <223> synthetic oligonucleotide
<400> 14
   acatgggata tggtactgac gac 23
<210> 15
   <211> 21
   <212> DNA
   <213> Artificial
<220>
   <223> synthetic oligonucleotide
<400> 15
   gcgaggcgct catgaacaag t 21
<210> 16
   <211> 21
   <212> DNA
   <213> Artificial
<220>
   <223> synthetic oligonucleotide
<400> 16
   cacggccacc atgaagtagc a 21
<210> 17
   <211> 22
   <212> DNA
   <213> Artificial
<220>
   <223> synthetic oligonucleotide
<400> 17
   gacacttgat gggctgaggt tc 22
<210> 18
   <211> 22
   <212> DNA
   <213> Artificial
<220>
   <223> synthetic oligonucleotide
<400> 18
   taagtcaggg gtgggagttt ac 22
<210> 19
   <211> 21
   <212> DNA
   <213> Artificial
<220>
   <223> synthetic oligonucleotide
<400> 19
   ctgcacgctg gtcttcctac t 21
<210> 20
   <211> 21
   <212> DNA
   <213> Artificial
<220>
   <223> synthetic oligonucleotide
<400> 20
   ccgatcttga ccatgagctt c 21
<210> 21
   <211> 23
   <212> DNA
   <213> Artificial
<220>
   <223> synthetic oligonucleotide
<400> 21
   tcagaaaccc ttcagtgcta cat 23
<210> 22
   <211> 23
   <212> DNA
   <213> Artificial
<220>
   <223> synthetic oligonucleotide
<400> 22
   atacacacgc agaaaccact ctt 23
<210> 23
   <211> 20
   <212> DNA
   <213> Artificial
<220>
   <223> synthetic oligonucleotide
<400> 23
   gtccccttct ccatctccag 20
<210> 24
   <211> 20
   <212> DNA
   <213> Artificial
<220>
   <223> synthetic oligonucleotide
<400> 24
   tatttgtgag ccagggcatt 20

## Description

### 2. BACKGROUND OF THE INVENTION

### 2.1. FIELD OF THE INVENTION

The present invention relates to compositions for treating and/or preventing synovial sarcoma. . Also, the present invention relates to methods, compositions and kits for diagnosing synovial sarcoma. Furthermore, the present invention relates to antibodies and their use in the treatment of synovial sarcoma.

### 2.2.. DESCRIPTION OF THE RELATED ART

Recently, molecular target therapy using humanized monoclonal antibodies such as trastuzumab (Herceptin) against ErbB2 (Fendly, B. M. et al., Cancer Res. 50: 1550-8., 1990) and rituximab (Rituxan) against CD20 (Maloney, D. G. et al., Blood. 90: 2188-95., 1997) has contributed to the improvement of treatment outcomes in some cases of breast cancer and malignant lymphoma. These promising therapies are the first examples of genomic research-based cancer drugs that bind directly to targeted proteins on the surface of tumor cells. The humanized antibodies are thought to exert an antitumor effect through inhibition of growth signal transduction by the blocking of the cell-surface receptor, and down-regulation of target molecules by interaction with specific antibodies and/or antibody-dependent cell-mediated cytotoxicity (ADCC). Although the precise mechanisms of the antibody-based antitumor effect remain to be elucidated, these therapies are promising alternatives, especially in the treatment of chemoresistant or radioresistant cancers.

Among sarcomas defined as malignant tumors occurring in the mesenchymal tissues, osteosarcomas, Ewing's sarcoma and rhabdomyosarcomas are generally sensitive to chemotherapy. Many other sarcomas, however, especially spindle cell sarcomas in adults, are difficult diseases to treat due to chemo- and radioresistance (Crist, W. M. et al., J Clin Oncol. 19: 3091-102., 2001; Wunder, J. S. et al., J Bone Joint Surg Am. 80: 1020-33., 1998; Ferguson, W. S. and Goorin, A. M., Cancer Invest. 19: 292-315., 2001; Adjuvant chemotherapy for localised resectable soft-tissue sarcoma of adults: meta-analysis of individual data. Sarcoma Meta-analysis Collaboration, Lancet. 350: 1647-54., 1997). Synovial sarcoma (SS) is a prototype of such tumors, and novel treatment modalities including antibody-based therapy should be developed for further improvement of outcomes, although the prognosis of SS has improved with advances in multidisciplinary treatment.

For the development of antibody-based therapy against target tumors, a critical key is identification of a cell-surface molecule that is overexpressed in the majority of the target tumors and whose expression is absent or minimal in the normal organ tissues. However, it is difficult to identify proteins specifically expressed in tumors, and there have been no reports of such proteins specifically expressed in synovial sarcoma and other tumors against which antibody-based therapies are desired to be established.

WO 02/055705 relates to nucleic acids and polypeptide sequences of the frizzled receptor family and methods of use thereof for treating and preventing diseases.

WO 02/092635 relates to compositions and methods for identifying, treating and preventing cancer by targeting the extracellular domains of the frizzled receptor family of proteins.

Koike, J et al., Biochem. Biophys. Res. Comm. 262:39-43, 1999 relates to the molecular cloning of frizzled-10.

WO 96/02641 relates to materials and methods relating to the diagnosis and prophylactic and therapeutic treatment of synovial sarcoma.

### 3. SUMMARY OF THE INVENTION

Considering the above problems and demands for developing therapeutics for synovial sarcoma, the group of the present inventors previously reported that SS may originate from neural crest cells and noted that the biological features of SS are similar to those of malignant peripheral nerve sheath tumor through genome-wide analysis of gene-expression patterns using a cDNA microarray consisting of 23,040 genes (Nagayama, S. et al., Cancer Res. 62: 5859-66., 2002). As a result, we have identified 26 genes that were commonly up-regulated in SS, whose products should be suitable molecular targets for the development of novel therapeutic drugs. Among these up-regulated genes, we selected a candidate gene suitable for the development of molecular target therapy for SS on the basis of the following criteria: (i) expression in the vital organs including brain, heart, lung, liver, kidney and bone marrow was relatively low to avoid critically adverse side effects; and (ii) the gene product has predicted to be the plasma integral membrane protein. Based on these criteria, we focused on one of the cell-surface receptors for the Wnts, Frizzled homologue 10 (FZD10), which belongs to the Frizzled family of seven-pass transmembrane proteins. Although expression of FZD10 has been demonstrated to be up-regulated in primary colorectal cancer (Terasaki, H. et al., Int J Mol Med. 9: 107-12., 2002) and primary gastric cancer (Kirikoshi, H. et al., Int J Oncol. 19: 767-71., 2001) as well as SS, the precise biological effects of FZD10 in tumorigenesis remain obscure. Hence, the possible role of FZD10 in tumor growth could potentially be elucidated by inhibition of signal transduction of FZD10.

At this time, the present inventors generated a specific polyclonal antibody (TT641 pAb) and monoclonal antibodies (mAbs) that recognized the N-terminal extracellular domain of FZD10 (FZD10-ECD) for the development of antibody-based therapy for synovial sarcoma (SS) . As the above-mentioned criteria for selection of molecular targets, the present inventors revealed that the expression of FZD10 was absent or low in normal vital organs except epithelia in some organ tissues with immunohistochemical analysis using TT641 pAb. Moreover, it was demonstrated by the present inventors that this specific antibody was effective in mediating antibody-dependent cell-mediated cytotoxicity (ADCC) against FZD10-overexpressing SS cells. In addition, *in vivo* experiments using nude mice successfully showed that intratumoral injection of TT641 pAb attenuated the growth of SS xenografts presumably through induction of apoptosis of tumor cells.

Based on the above findings, the present inventors concluded that the antibody for FZD10 has therapeutic potential in the treatment and diagnosis of SS and other FZD10-overexpressing tumors.

The present invention provides:
1. An antibody against Frizzled homologue 10 (FZD10) protein or a fragment thereof for use in a method of treating or preventing synovial sarcoma.
2. An *in vitro* method for diagnosis or prognosis of synovial sarcoma, or of a predisposition to developing synovial sarcoma in a subject, comprising:
   (a) contacting a sample from the subject with an antibody against FZD10 protein or a fragment thereof;
   (b) detecting the FZD10 protein in the sample; and
   (c) judging whether or not the subject suffers from or is at risk of developing synovial sarcoma based on the relative abundance of the FZD10 protein compared to a control.
3. A pharmaceutical composition for use in treating or preventing synovial sarcoma, comprising an antibody or a fragment that is raised against a peptide comprising at least at least residues 43-56, 61-72, 156-169, 157-170, 157-172, 161-173, 174-191, 189-202, 214-225, or 1-225 of an amino acid sequence shown in SEQ ID NO: 1, and a pharmaceutically acceptable carrier or excipient, wherein said antibody or antibody fragment specifically recognise FZD10 and does not recognise other members of the FZD family.
4. A kit for diagnosis or prognosis of synovial sarcoma, comprising an antibody or a fragment that is raised against a peptide comprising at least residues 43-56, 61-72, 156-159, 157-170, 157-172, 161-173, 174-191, 189-202, 214-225, or 1-225 of the amino acid sequence shown in SEQ ID NO: 1, wherein said antibody or antibody fragment specifically recognise FZD10 and does not recognise other members of the FZD family.
5. An antibody or an antibody fragment against Frizzled homologue 10 (FZD10) protein, which is raised against a peptide comprising at least residues 43-56, 61-72, 156-169, 157-170, 157-172, 161-173, 174-191, 189-202, 214-225, or 1-225 of an amino acid sequence shown in SEQ ID NO: 1, wherein said antibody or antibody fragment specifically recognise FZD10 and does not recognise other members of the FZD family. The present invention further provides:
6. The antibody of 1 or the method of 2, wherein the antibody is polyclonal or monoclonal antibody.
7. The antibody of 1, wherein the antibody is raised against a peptide comprising at least residues 43-56, 61-72, 156-169, 157-170, 157-172, 161-173, 174-191, 189-202, 214-225, or 1-225 of an amino acid sequence shown in SEQ ID NO: 1.
8. The method of 2, wherein the antibody is raised against a peptide comprising at least residues 43-56, 61-72, 156-169, 157-170, 157-172, 161-173, 174-191, 189-202, 214-225, or 1-225 of an amino acid sequence shown in SEQ ID NO: 1.
9. The antibody or a fragment thereof of 5, which is raised against a peptide consisting of residues 1-225 of the amino acid sequence shown in SEQ ID NO: 1.
10. The antibody or a fragment thereof of 5 or 9, which is a polyclonal or monoclonal antibody.

### 4. BRIEF DESCRIPTION OF THE DRAWINGS

Fig. 1A is a photograph showing a northern blot analysis of FZD10 in normal human adult tissues (heart, brain, lung, liver, kidney, pancreas, bone marrow, and placenta), SS cell lines (HS-SY-2 and YaFuSS) and surgical SS specimens (SS487 and SS582).
Fig. 1B is a photograph showing a northern blot analysis of FZD10 in colon cancer cell lines (LoVo, HT29 and SW480) and SS cell lines (SYO-1, HS-SY-2, and YaFuSS).
Figs. 2A and 2B are photographs showing the specificity of the affinity-purified FZD10-ECD antibody (TT641 pAb) that recognized the N-terminal extracellular domain of FZD10 (FZD10-ECD).

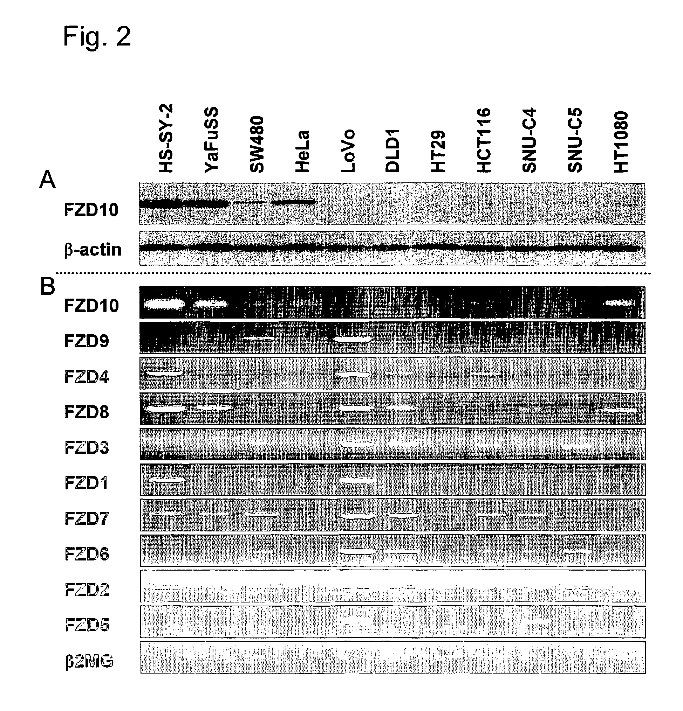

Fig. 2A are photographs showing western blot analyses of FZD10 in several tumor cell lines: SS cell lines (HS-SY-2 and YaFuSS), colon cancer cell lines (SW480, LoVo, DLD1, HT29, HCT116, SNU-C4 and SNU-C5), cervical adenocarcinoma cell line (HeLa) and fibrosarcoma cell line (HT1080) . β-actin expression was used as a loading control.
Fig. 2B are photographs showing semi-quantitative RT-PCR analysis of the FZD gene family in the same tumor cell lines as those examined in Fig. 2A. Expression of β2-microglobulin gene β2MG) served as an internal control. FZD family members were placed in order of the homology of amino acid sequences to FZD10-ECD; FZD9 was the most homologous to FZD10-ECD.

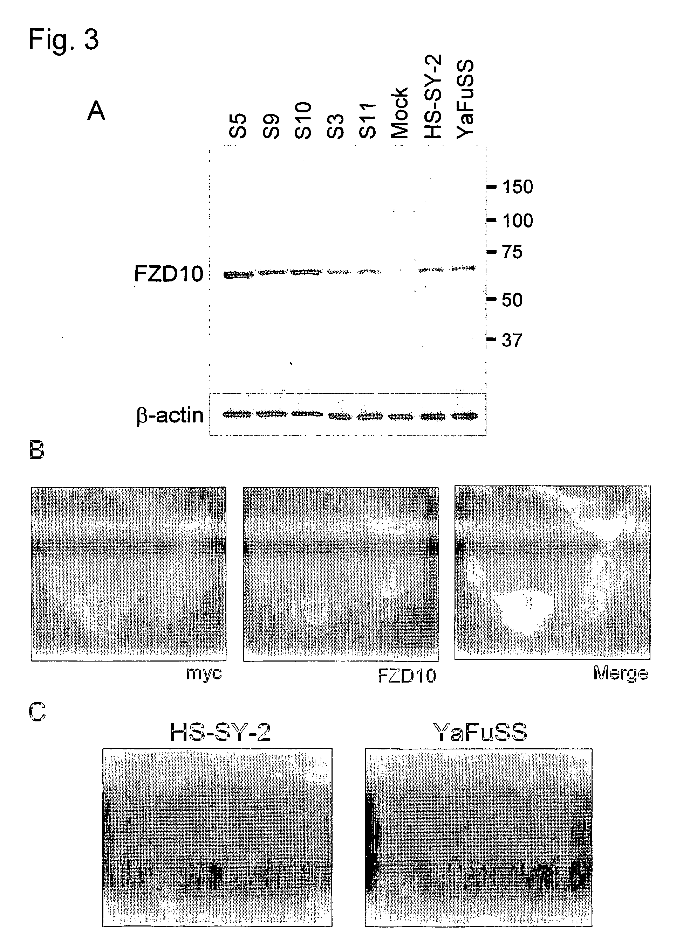

Figs. 3A, 3B and 3C are photographs showing subcellular localization of FZD10 protein with immunocytochemical analysis.
Fig. 3A is a photograph showing western blots indicating the establishment of COS7-FZD10 cells, which stably overexpressed FZD10. S5, S9, S10, S3 and S11 were representatives of COS7-FZD10 stable transfectant cells, and exogenously expressed products were the same size as endogenous FZD10 expressed in the SS cell lines, HS-SY-2 and YaFuSS.
Fig. 3B a photograph showing an immunocytochemical staining using anti-myc antibody and the TT641 pAb. The left panel shows a cell immunostained with Texas Red-conjugated anti-myc. The middle panel shows the same cell treated with TT641 pAb (Alexa Flour 488), and both signals are overlaid in the double-color image as a yellow signal (right panel).
Fig. 3C are photographs showing an immunocytochemical staining with TT641 pAb for the detection of endogenous FZD10 in SS cell lines (HS-SY-2 and YaFuSS) .

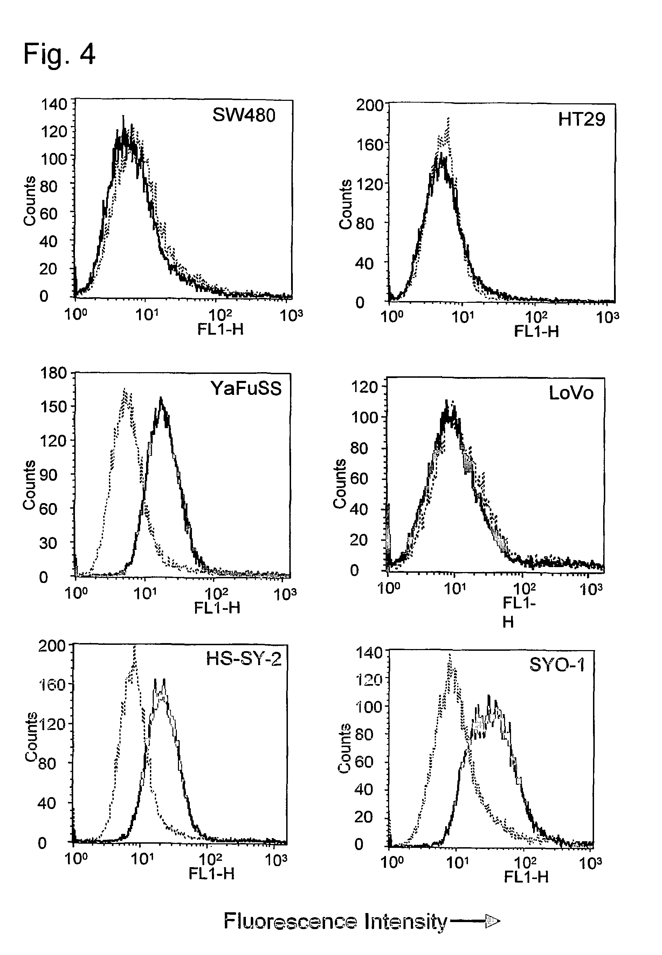

Fig. 4 are graphs showing a flow cytometric analysis using TT641 pAb in several tumor cell lines. Solid lines show the expression level of FZD10, a cell-surface antigen, detected with TT641 pAb, whereas broken lines depict the fluorescent signal of cells incubated with non-immunized rabbit IgG as a negative control.

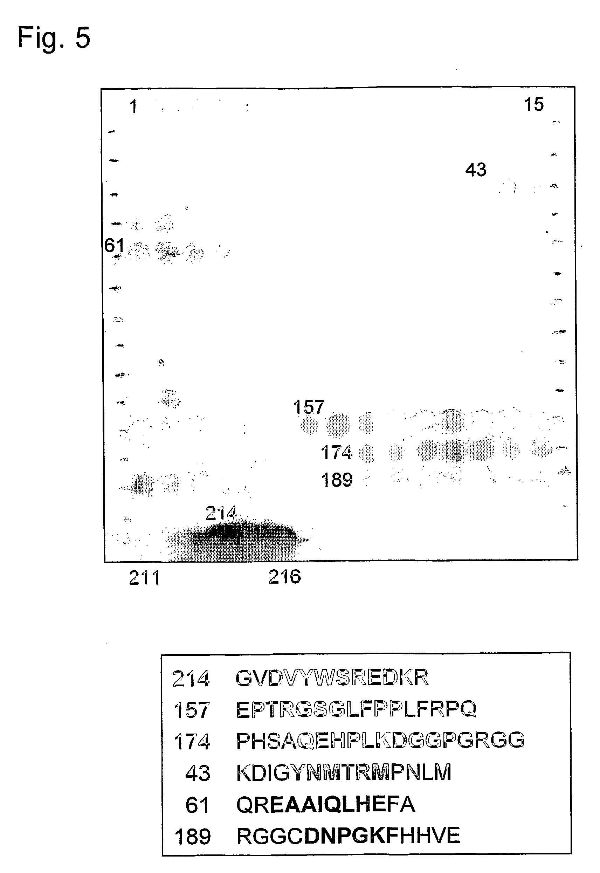

Fig. 5 is a photograph showing an epitope mapping of TT641 pAb with synthetic overlapping linear peptides. The membrane containing a series of 10-residue peptides differing by one amino acid and covering the entire amino acid of FZD10-ECD was probed with TT641 pAb, and the binding was detected with HRP-conjugated goat anti-rabbit IgG. Bold letters indicate the possible core epitopes of FZD10-ECD.

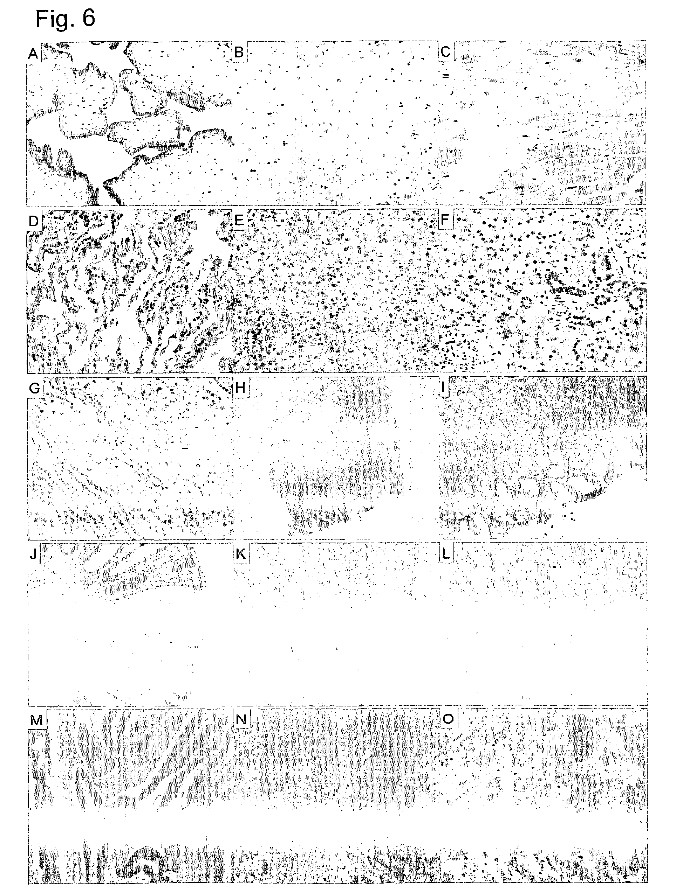

Figs. 6A to 6O are photographs showing immunohistochemical analysis using the TT641 pAb in normal adult human tissues, SS tumor tissues, primary colon cancer, and metastatic liver lesions of the colon cancer. In these figures, A = placenta, B = brain, C = heart, D = lung, E = liver, F and G = kidneys from different individuals, H and I = stomach from the same individual, J = colon, K and L = SS tumor cells of the same biphasic SS specimen, and M, N, and O = primary colon cancer and its metastatic liver lesion of the same patient. The original magnification of A*_{F}* B*_{F}* C*_{F} D_{F}* E*_{F}* F*_{F} G_{F}* I*_{F}* J*_{F}* K*_{F}* and M = x 100; H and N = x 40; L and O = x 200.

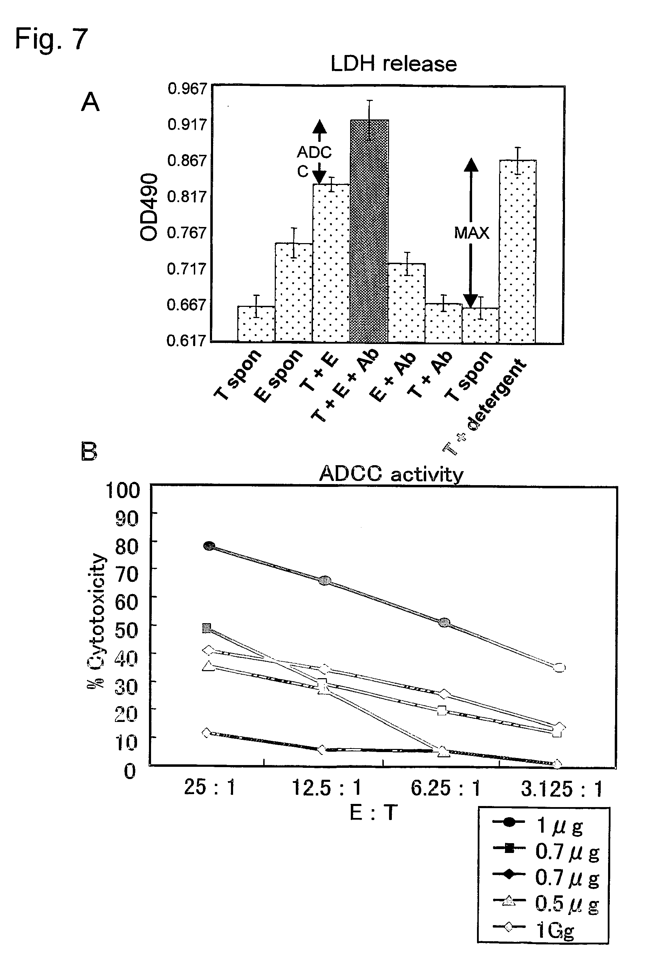

Fig. 7A is a graph showing TT641 pAb mediated ADCC against FZD10- overexpressing cells. Cytotoxicity was assayed by quantitative measurement of released lactate dehydrogenase (LDH) upon cell lysis, when target and effector cells were co incubated with 7 µg/ml of TT641 pAb at an E:T ratio of 25:1. In this figure, T = target cells (SYO-1), E = effector cells (PBMCs), T spon = spontaneous release of LDH from target cells, E spon = spontaneous release of LDH from effector cells, and Ab = TT641 pAb.
Fig. 7B is a graph showing that TT641 pAb mediated ADCC against FZD10- overexpressing cells. Cell-mediated cytotoxicity against FZD10-overexpressing cells at several E:T ratios, indicating positive correlation with the amount of TT641 pAb added to the medium.

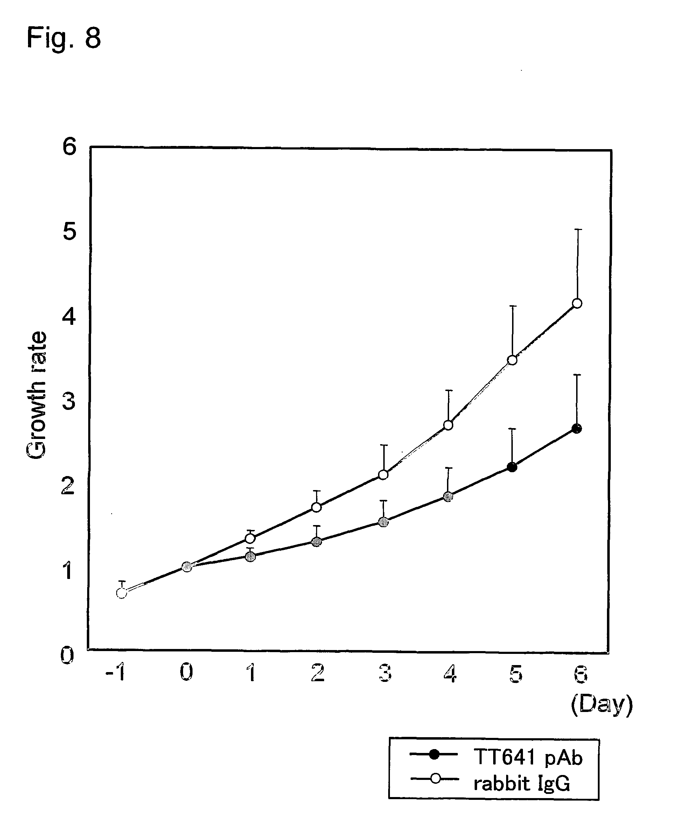

Fig. 8 is a graph showing that TT641 pAb exerted a growth inhibitory effect on SS xenografts. The tumor growth was assessed as the ratio of tumor volume at the indicated day to that calculated at the initial day of treatment in the experimental group (n=16) treated with TT641 pAb (closed circle) and in the control group (n=15) with non-immunized rabbit IgG (open circle) . Treatment was continued for 5 consecutive days (Days 0-4). Data was expressed as mean + SD.

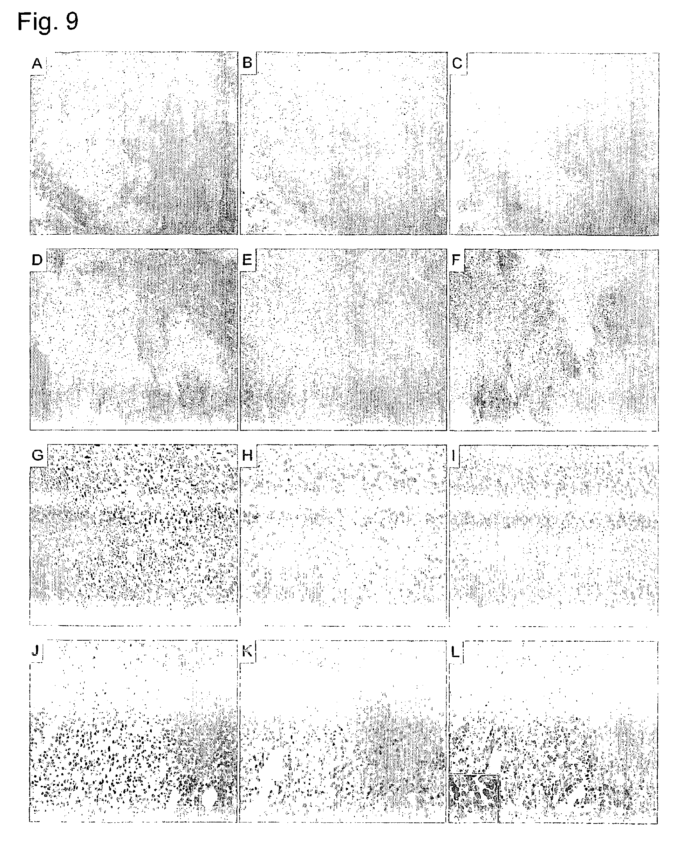

Fig. 9A to 9L are photographs showing TUNEL analysis and immunohistochemical staining for Ki-67. At 2 days after the completion of treatment, tumors were extirpated and fixed in 10% formaldehyde. TUNEL analysis and immunohistochemical staining for Ki-67, a reliable indicator of cell proliferation ability, were performed on the serial sections of paraffin-embedded specimens from tumors treated with non-immunized rabbit antibody (A, B, C, G, H and I) and TT641 pAb (D, E, F, J, K and L) . In this figures, A, D, G and J = HE staining; B, E, H and K = immunostaining for Ki-67, and C, F, I and L = TUNEL analysis. The original magnification of A-F = x 40; G-L = x 200; inset in L = x 400.

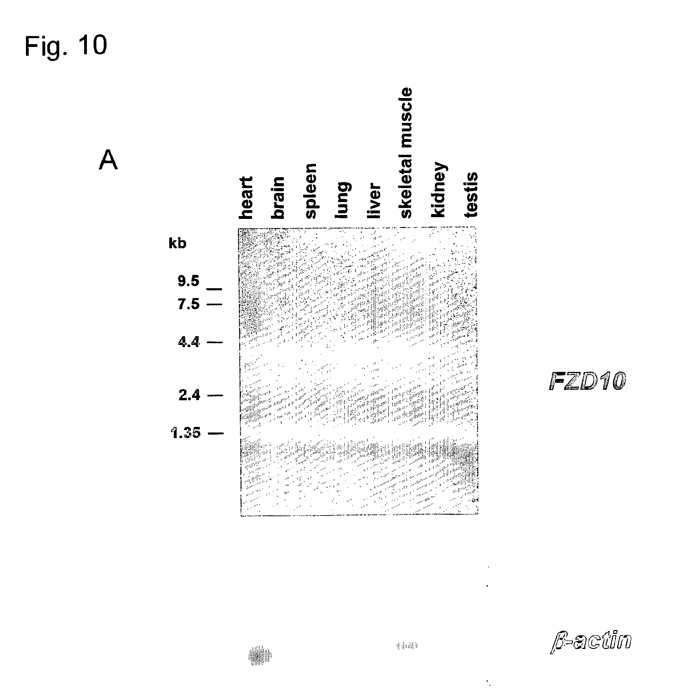

Fig. 10A is a photograph showing expression of mouse *FZD10* in normal mouse tissues detected by northern blot analysis of FZD10 in eight normal mouse tissues (heart, brain, spleen, lung, liver skeletal muscle, kidney or testis). Expression of β-actin was used as a loading control.

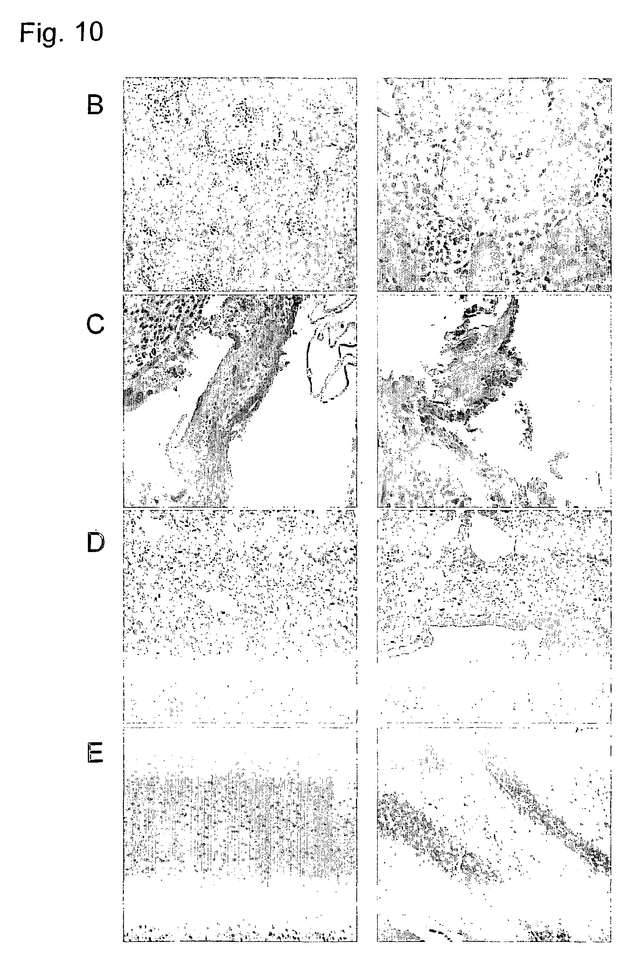

Figs. 10B to 10E are photographs showing immunocytochemical staining in normal mouse kidney (B), placenta (C), lung (D), and brain (E) tissues using the TT641 pAb.

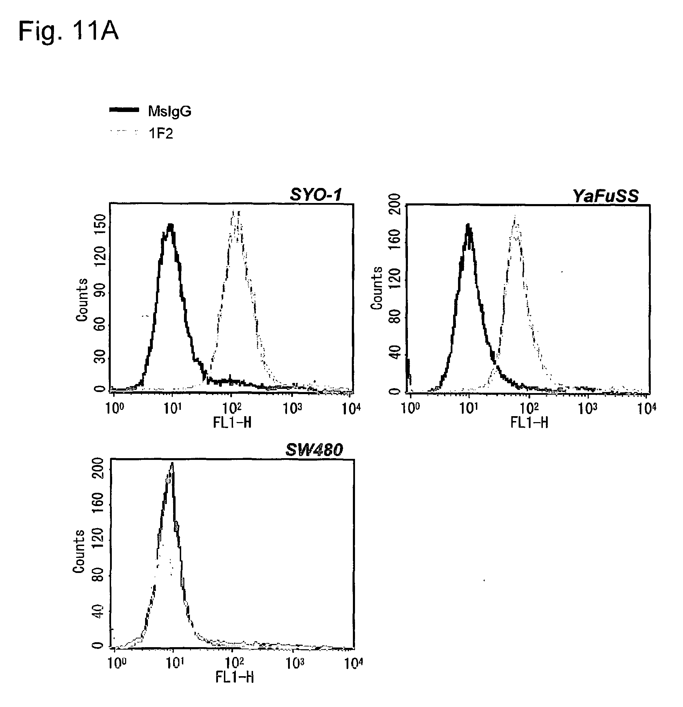

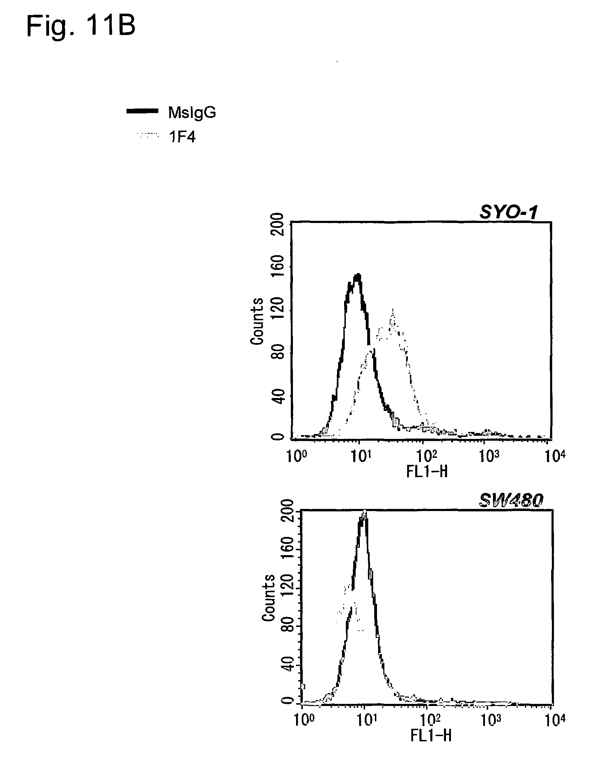

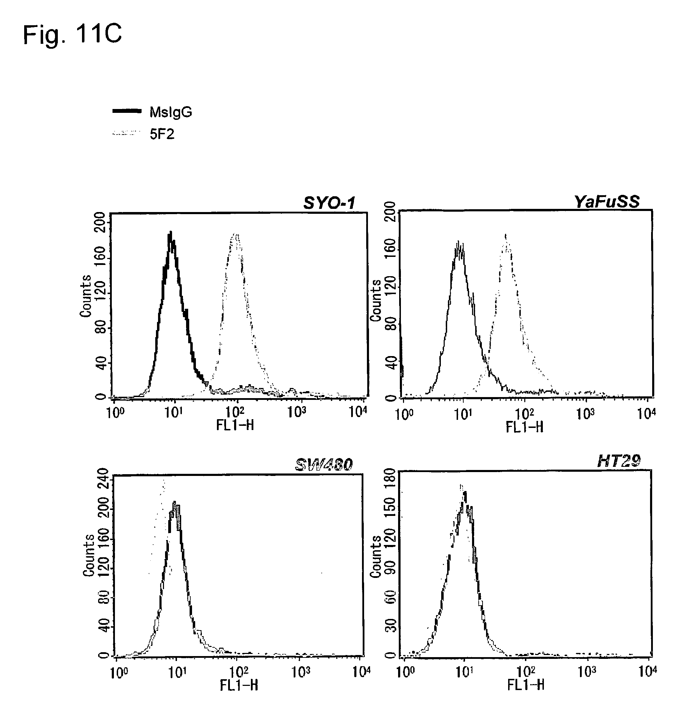

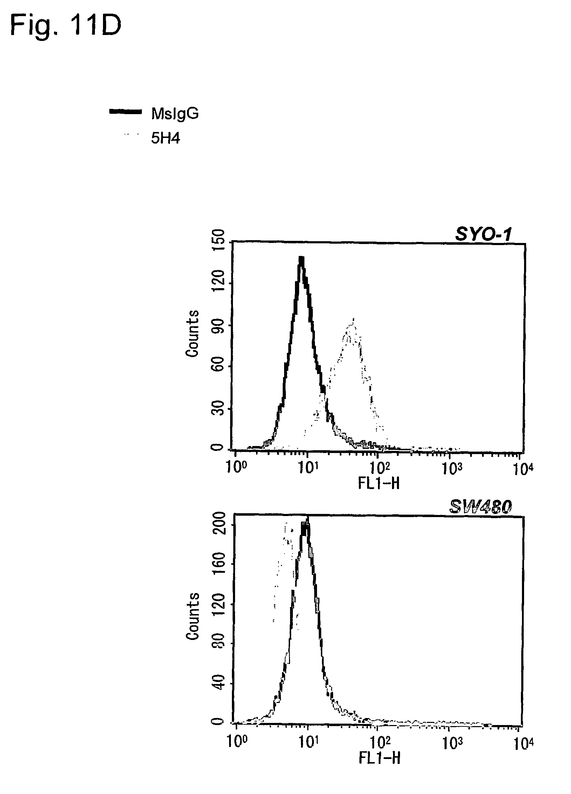

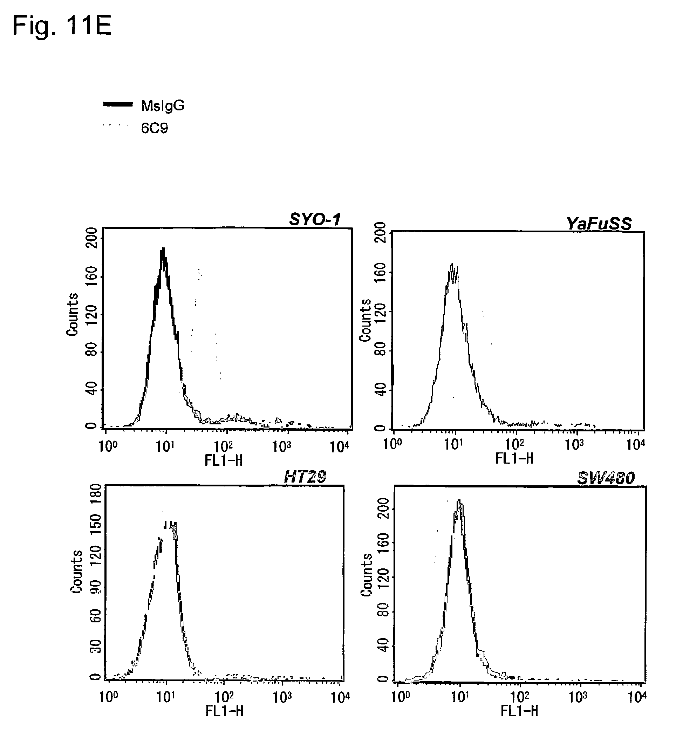

Figs. 11A to 11E are graphs showing flow-cytometric analysis using anti-FZD10 mAbs, 1F2 (A), 1F4 (B), 5F2 (C), 5H4 (D) and 6C9 (E), in SS lines (SYO-1 and YaFuSS; upper panels) and colon-cancer cell lines (SW480 and HT29; lower panels) . Gray lines show expression of the cell-surface antigen FZD10 detected by each mAb; black lines depict the fluorescent signals of cells incubated with non-immunized rabbit IgG as a negative control.

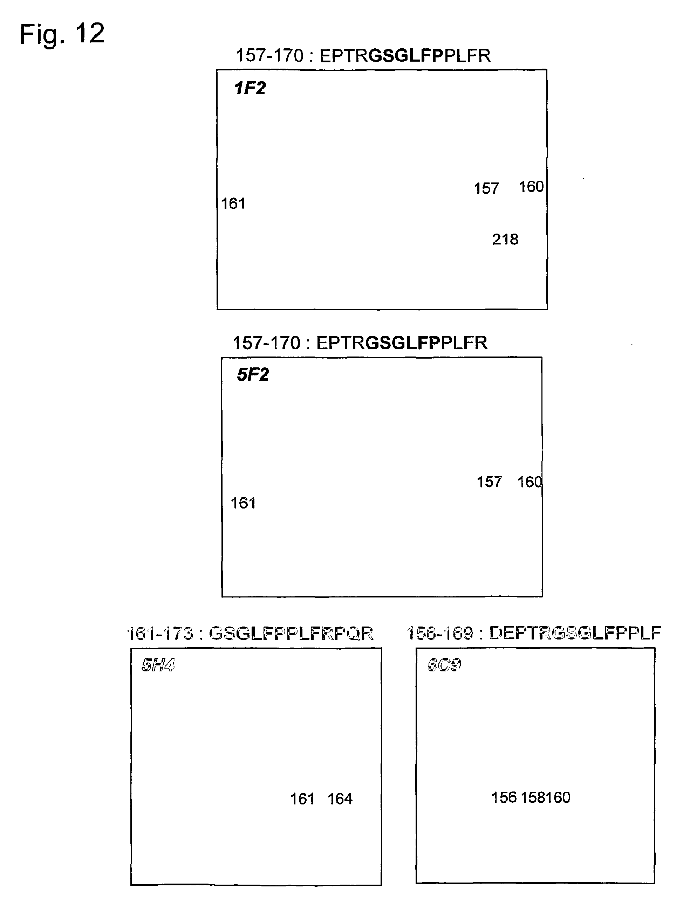

Fig. 12 are photographs showing epitope mapping of mAbs, 1F2, 5F2, 5H4 and 6C9 with synthetic overlapping linear peptides.

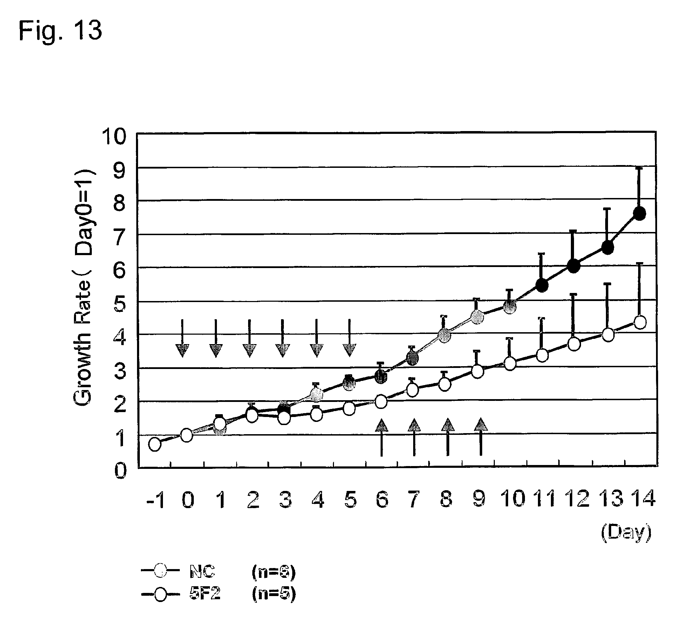

Fig. 13 is a graph showing growth-inhibitory effect of mAb 5F2 on SS xenografts. Tumor growth was assessed as the ratio of tumor volume at the indicated day to that calculated on the initial day of treatment in the experimental group (5F2, n=5) treated with mAbs (5F2, open circles) and in the control group (n=6) given non-immunized rabbit IgG (closed circles). Treatment was continued for ten consecutive days (Days 0-9; arrows). Data are expressed as means + SD.

### 5. DETAILED DESCRIPTION AND PREFERRED EMBODIMENTS OF THE INVENTION

The present invention relates to a method for treating and/or preventing a disease which is associated with Frizzled homologue 10 (FZD10) (FZD10-associated disease) in a subject and to a method for diagnosis and/or prognosis of the disease. Compositions useful in these methods are also encompassed within the scope of the present invention.

The present invention is based on the findings that the specific polyclonal antibody for FZD10 (TT641 pAb) and the monoclonal antibodies for FZD10 (mAbs) are effective in mediating antibody-dependent cell-mediated cytotoxicity (ADCC) against FZD10-overexpressing SS cells, and in inhibiting the growth of SS xenografts, as described below. Also, the present invention is based on the findings that FZD10 is specifically expressed in certain tumors including synovial sarcoma, and that these tumors can be detected using the specific antibody for FZD10.

### 5.1. PRODUCTION OF AN ANTIBODY

Antibodies that can be used in the present invention specifically react against an FZD10 protein derived from an FZD10-associated disease. The term "antibody" used herein means an antibody molecule as a whole, or its fragments such as Fab fragments, F(ab')₂ fragments and. Fv fragments, which can bind to the protein or its partial peptides as the antigen. The antibody can be either a polyclonal antibody or a monoclonal antibody. It can also be a humanized or chimeric antibody, or a single chain Fv (scFv) antibody. The antibodies (polyclonal antibodies and monoclonal antibodies) for use in the present invention can be prepared, for example, by the following process.

### 5.1.1. IMMUNOGEN (ANTIGEN)

Initially, a protein for use as an immunogen (antigen) is prepared for the preparation of an antibody useful in the present invention. FZD10 protein or its partial peptide is used as an immunogen protein. The amino acid sequence of FZD10 protein used as the immunogen in the present invention and the cDNA sequence encoding the protein are publicly available in GenBank as Accession Nos. BAA84093 (SEQ ID NO: 1) and AB027464 (SEQ ID NO: 2), respectively. The FZD10 protein or its partial peptide for use as the immunogen can be synthetically prepared according to a procedure known in the art such as a solid-phase peptide synthesis process, using the available amino acid sequence information. The partial peptide of FZD10 protein use as an immogen is selected from include, a peptide containing residues 1-225 of the amino acid sequence shown in SEQ ID NO: 1, which corresponds to the N-terminal extracellular domain of FZD10 protein (FZD10-ECD), peptides containing residues 43-56, 61-72, 156-169, 157-170, 157-172, 161-173, 174-191, 189-202, or 214-225 of the FZD10 protein (SEQ ID NO: 1) or at least five residues, and preferably six to ten residues, of these partial sequences. . A peptide containing the residues 214-225 of the FZD10 protein (SEQ ID NO: 1) is preferably used as the immunogen in the present invention.

The protein or its partial peptide can also be prepared by using the sequence information of cDNA encoding FZD10 protein or its partial peptide according to a known gene recombination procedure. The production of the protein or its partial peptide according to such a gene recombination procedure will be illustrated below.

A recombinant vector for the production of protein can be obtained by linking the above cDNA sequence to an appropriate vector. A transformant can be obtained by introducing the recombinant vector for the production of protein into a host so that the target FZD10 protein or its partial peptide can be expressed.

As the vector, a phage or plasmid that is capable of autonomously replicating in a host is used. Examples of a plasmid DNA include pET28, pGEX4T, pUC118, pUC119, pUC18, pUC19, and other plasmid DNAs derived from *Escherichia coli;* pUB110, pTP5, and other plasmid DNAs derived from *Bacillus subtilis;* and YEp13, YEp24, YCp50 and other plasmid DNAs derived from yeast. Examples of a phage DNA include lambda phages such as λgt11 and λZAP. In addition, animal virus vectors such as retrovirus vector and vaccinia virus vector can be used, and insect virus vectors such as baculovirus vector can also be used.

The DNA encoding the FZD10 protein or its partial peptide (hereinafter referred to as FZD10 DNA) is inserted into the vector, for example, by the following method. In this method, purified DNA is cleaved by an appropriate restriction enzyme and inserted into a restriction enzyme site or a multi-cloning site of an appropriate vector DNA to ligate into the vector.

In addition to a promoter and the FZD10 DNA, any of enhancers and other *cis* elements, splicing signals, poly A addition signals, selective markers, ribosome binding site (RBS), and other elements can be ligated into the recombinant vector for the production of protein for use in mammalian cells, if desired.

For ligating the DNA fragment to the vector fragment, a known DNA ligase can be used. The DNA fragment and the vector fragment are annealed and ligated, thereby producing a recombinant vector for the production of a protein.

The host for use in transformation is not specifically limited as long as it allows the FZD10 protein or its partial peptide to be expressed therein. Examples of the host include bacteria, for example, *E. coli,* and Bacillus; yeast, for example, *Saccharomyces cerevisiae;* animal cells, for example, COS cells, Chinese Hamster Ovary (CHO) cells, and insect cells.

For example, when a bacterium is used as the host, the recombinant vector for the protein production should preferably be capable of autonomously replicating in the host bacterium and comprise a promoter, a ribosome binding site, the FZD10 DNA, and a transcription termination sequence. The recombinant vector may further comprise a gene for regulating the promoter. An example of *Escherichia coli* includes *Escherichia coli* BRL, and an example of Bacillus is *Bacillus subtilis.* Any promoter that can be expressed in the host such as *Escherichia coli* can be used herein.

The recombinant vector can be introduced into the host bacterium by any procedures known in the art. Such procedures include, for example, a method using calcium ions and an electroporation.

When yeast cell, an animal cell, or an insect cell is used as the host, a transformant can be produced according to a known procedure in the art, and then the FZD10 protein or its partial peptide can be produced in the host (transformant).

The FZD10 protein or its partial peptide for use as the immunogen in the present invention can be obtained from a culture of the above-generated transformant. The "culture" refers to any of culture supernatant, cultured cells, cultured microorganisms, and homogenates thereof. The transformant is cultured in a culture medium by a conventional process of culturing a host.

The culture medium for culturing the transformant obtained by using *Escherichia coli,* yeast, or other microorganisms as the host can be either a natural medium or a synthetic medium, as long as it comprises a carbon source, nitrogen source, inorganic salts, and other components utilizable by the microorganism and enables the transformant to grow efficiently.

The transformant is generally cultured by shaking culture or aeration culture with stirring under aerobic conditions at 25 °C to 37 °C for 3 to 6 hours. During culturing, pH is held at a level near neutrality by adjustment with, for example, an inorganic or organic acid, and an alkaline solution. During culturing, antibiotics such as ampicillin or tetracycline may be added to the medium according to the selective marker inserted into the recombinant expression vector, if necessary.

After culturing, when the FZD10 protein or its partial peptide is produced within the microorganism or cell, the protein or its partial peptide is extracted by homogenizing the microorganism or cell. When the FZD10 protein or its partial peptide is secreted from the microorganism or cell, the culture medium is used as is, or debris of the microorganism or cell is removed from the culture medium, for example, by centrifugation. Thereafter, the FZD10 protein or its partial peptide can be isolated from the culture and purified by a conventional biochemical method for the isolation and purification of proteins, such as ammonium sulfate precipitation, gel chromatography, ion-exchange chromatography, and affinity chromatography, either individually or in combination.

Whether or not the FZD10 protein or its partial peptide has been obtained can be confirmed, for example, by SDS polyacrylamide gel electrophoresis.

Next, the obtained FZD10 protein or its partial peptide is dissolved in a buffer to prepare an immunogen. Where necessary, an adjuvant can be added thereto for effective immunization. Such adjuvants include, for example, commercially available Freund's complete adjuvant and Freund's incomplete adjuvant. Any of these adjuvants can be used alone or in combination.

### 5.1.2. POLYCLONAL ANTIBODY

To prepare a polyclonal antibody, the immunogen prepared in Section 5.1.1. above is administered to a mammal such as a rabbit, rat, or mouse. An adjuvant such as Freund's complete adjuvant (FCA) or Freund's incomplete adjuvant (FIA) may be used according to necessity. The immunization is performed mainly by intravenous, subcutaneous, or intraperitoneal injection. The interval of immunization is not specifically limited and the mammal is immunized one to 7 times at the intervals of several days to several weeks. The antibody titer is determined 1 to 7 days after the last immunization, for example, by enzyme-linked immunosorbent assay (ELISA), enzyme immunoassay (EIA), or radioimmunoassay (RIA) . Blood is collected on the day when the maximum antibody titer is measured, to obtain an antiserum. Thereafter, the reactivity of the polyclonal antibody in the antiserum is measured, for example, by ELISA.

### 5.1.3. MONOCLONAL ANTIBODY

The immunogen prepared in Section 5.1.1. above is administered to a mammal such as a rabbit, rat, or mouse. An adjuvant such as Freund's complete adjuvant (FCA) or Freund's incomplete adjuvant (FIA) may be used as necessary. The immunization is performed mainly by intravenous, subcutaneous, or intraperitoneal injection. The interval of immunization is not specifically limited and the mammal is immunized one to 3 times at intervals ranging from several days to weeks. Antibody-producing cells are collected 1 to 7 days after the last immunization. Examples of the antibody-producing cells include pancreatic cells, lymph node cells, and peripheral blood cells.

To obtain a hybridoma, an antibody-producing cell and a myeloma cell are fused. As the myeloma cell to be fused with the antibody-producing cell, a generally available established cell line can be used. Preferably, the cell line used should have drug selectivity and properties such that it can not survive in a HAT selective medium (containing hypoxanthine, aminopterin, and thymidine) in unfused form and can survive only when fused with an antibody-producing cell. Possible myeloma cells include, for example, mouse myeloma cell lines such as P3X63-Ag.8.U1 (P3U1), and NS-I.

Next, the myeloma cell and the antibody-producing cell are fused. For the fusion, these cells are mixed, preferably at the ratio of the antibody-producing cell to the myeloma cell of 5:1, in a culture medium for animal cells which does not contain serum, such as DMEM and RPMI-1640 media, and fused in the presence of a cell fusion-promoting agent such as polyethylene glycol (PEG). The cell fusion may also be carried out by using a commercially available cell-fusing device using electroporation.

Then, the hybridoma is picked up from the cells after above fusion treatment. For example, a cell suspension is appropriately diluted with, for example, the RPMI-1640 medium containing fetal bovine serum and then plated onto a microtiter plate. A selective medium is added to each well, and the cells are cultured with appropriately replacing the selective medium. As a result, the cells that grow about 30 days after the start of culturing in the selective medium can be obtained as the hybridoma.

The culture supernatant of the growing hybridoma is then screened for the presence of an antibody that reacts with the FZD10 protein or its partial peptide. The screening of hybridoma can be performed according to a conventional procedure, for example, using enzyme-linked immunosorbent assay (ELISA), enzyme immunoassay (EIA) or radioimmunoassay (RIA) . The fused cells are cloned by the limiting dilution to establish a hybridoma, which produces the monoclonal antibody of interest.

The monoclonal antibody can be collected from the established hybridoma, for example, by a conventional cell culture method or by producing the ascites. If necessary, the antibody can be purified in the above-described antibody collecting method according to a known procedure such as ammonium sulfate precipitation, ion-exchange chromatography, gel filtration, affinity chromatography, or a combination thereof.

The globulin type of the monoclonal antibodies useful in the present invention is not specifically limited, as long as they are capable of specifically binding to the FZD10 protein and can be any of IgG, IgM, IgA, IgE, and IgD. Among them, IgG and IgM are preferred.

### 5.1.4. OTHER ANTIBODIES

In addition, techniques developed for the production of "chimeric antibodies" (Morrison et al., 1984, Proc. Natl. Acad. Sci., 81: 6851-6855; Neuberger et al., 1984, Nature, 312: 604-608; Takeda et al., 1985, Nature, 314: 452-454) can be used. These involve splicing the genes from a mouse antibody molecule of appropriate antigen specificity together with genes from a human antibody molecule of appropriate biological activity. A chimeric antibody is a molecule in which different portions are derived from different animal species, such as those having a variable region derived from a murine mAb and a human immunoglobulin constant region, e. g., "humanized antibodies."

Alternatively, techniques described for the production of single chain antibodies (U.S. Patent No. 4,946,778; Bird, 1988, Science 242: 423-426; Huston et al., 1988, Proc. Natl. Acad. Sci. USA 85: 5879-5883; and Ward et al., 1989, Nature 334: 544-546) can be adapted to produce single chain antibodies against FZD10 protein or a peptide thereof. Single chain antibodies are formed by linking the heavy and light chain fragments of the Fv region via an amino acid bridge, resulting in a single chain polypeptide. Techniques for the assembly of functional Fv fragments in E. coli may also be used (Skerra et al., 1988, Science 242: 10381041).

### 5.1.5. ANTIBODY FRAGMENTS

Antibody fragments that specifically recognize a portion (epitope) of a protein of interest may be generated by known techniques. For example, such fragments include, but are not limited to the F (ab')₂ fragments that can be produced by pepsin digestion of the antibody molecule and the Fab fragments that can be generated by reducing the disulfide bridges of the F (ab')₂ fragments.

Alternatively, Fab expression libraries may be constructed (Huse et al. , 1989, Science, 246: 1275-1281) to allow rapid and easy identification of monoclonal Fab fragments with the desired specificity.

### 5.2. THERAPEUTIC USES

Described below are methods and pharmaceutical compositions for treating and/or preventing synovial sarcoma using the antibody of the present invention. The outcome of a treatment is to at least produce in a treated subject a healthful benefit, which in the case of tumors, includes but is not limited to remission of the tumors, palliation of the symptoms of the tumors, and control of metastatic spread of the tumors.

Specifically, the method for treating and/or preventing FZD10-associated disease in a subject according to the present invention comprises administering to a subject in need thereof the antibody or the fragment described above (see, Section 5.1.).

The term "subject" herein refers to a subject who has suffered from FZD10-associated disease and also a subject suspected to have FZD10-associated disease. The subject in the present invention may be animals including mammals and avian animal. For example, mammals may include humans, mice, rats, monkeys, rabbits, and dogs.

The term "FZD10-associated disease" herein refers to a disease associated with the over-expression of FZD10 protein. Specifically, the FZD10-associated disease is synovial sarcoma (SS).

### 5.2.1. PHARMACEUTICAL COMPOSITIONS

The antibody described herein can be administered to a subject at effective doses to treat or prevent synovial sarcoma. An effective dose refers to that amount of an antibody sufficient to result in a healthful benefit in the treated subject. Formulations and methods of administration that can be employed when the pharmaceutical composition contains an antibody of the present invention are described below.

Pharmaceutical compositions for use in accordance with the present invention can be formulated in conventional manner using one or more pharmaceutically acceptable carriers or excipients.

The antibodies can be formulated for parenteral administration (i. e., intravenous or intramuscular) by injection, via, for example, bolus injection or continuous infusion. Formulations for injection can be presented in unit dosage form, e.g., in ampoules or in multi-dose containers, with an added preservative. The compositions can take such forms as suspensions, solutions, or emulsions in oily or aqueous vehicles, and can contain formulatory agents such as suspending, stabilizing and/or dispersing agents. Alternatively, the antibody can be in lyophilized powder form for constitution with a suitable vehicle, e.g., sterile pyrogen-free water, before use.

### 5.2.2. DOSE AND ADMINISTRATION ROUTE

Toxicity and therapeutic efficacy of the antibody of the present invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD/ED.

Antibodies that exhibit large therapeutic indices are preferred. While antibodies that exhibit toxic side effects can be used, care should be taken to design a delivery system that targets such antibodies to the site of affected tissue in order to minimize potential damage to uninfected cells and, thereby, reduce side effects.

The data obtained from the cell culture assays and animal studies can be used in formulating a range of dosages for use in humans. The dosage of such antibodies lies preferably within a range of circulating plasma concentrations that include the ED50 with little or no toxicity. The dosage can vary within this range depending upon the dosage form employed and the route of administration utilized. For any antibody used in the method of the invention, the effective dose can be estimated initially from cell culture assays. A dose can be formulated in animal models to achieve a circulating plasma concentration range that includes the IC50 (i.e., the concentration of the test antibody that achieves a half-maximal inhibition of symptoms) as determined in cell culture. Such information can be used to more accurately determine useful doses in humans. Levels in plasma can be measured, for example, by high performance liquid chromatography.

While depending on the conditions and age of the subject and/or administration route, one skilled in the art can select an appropriate dose of the pharmaceutical composition of the present invention. For example, the pharmaceutical composition of the present invention is administered in an amount such that the antibody according to the present invention is administered to the subject in a day in an amount of about 3 to about 15 µg per kg body weight of subject, and preferably of about 10 to about 15 µg per kg body weight of subject. The administration interval and times can be selected in consideration of the condition and age of the subject, administration route, and response to the pharmaceutical composition. For example, the pharmaceutical composition can be administered to the subject one to 5 times, preferably 1 times a day for 5 to 10 days.

The pharmaceutical composition can be administered systemically or locally. It is preferably administered in a targeting delivery manner so as to deliver the active component to an affected site.

### 5.2.3. COMBINATION THERAPY

In particular embodiments, the methods and compositions of the present invention are used for the treatment or prevention of synovial sarcoma together with one or a combination of chemotherapeutic agents including, but not limited to, methotrexate, taxol, mercaptopurine, thioguanine, cisplatin, carboplatin, mitomycin, bleomycin, doxorubicin, idarubicin, daunorubicin, dactinomycin, vinblastine, vincristine, vinorelbine, paclitaxel, and docetaxel.

With respect to radiation therapy, any radiation therapy protocol can be used depending upon the type of FZD10-associated disease to be treated. For example, but not by way of limitation, X-ray radiation can be administered. Gamma ray emitting radioisotopes, such as radioactive isotopes of radium, cobalt, and other elements may also be administered to expose tissues.

In another embodiment, chemotherapy or radiation therapy is administered, preferably at least an hour, five hours, 12 hours, a day, a week, a month, and more preferably several months (e. g., up to three months) subsequent to using the methods and compositions containing the antibody of the present invention. The chemotherapy or radiation therapy administered prior to, concurrently with, or subsequent to the treatment using the methods and compositions according to the present invention can be administered by any method known in the art.

### 5.3. DIAGNOSTIC AND PROGNOSTIC USES

Antibodies directed against FZD10 protein or peptide fragments thereof in Section 5.1.2. may also be used as diagnostics and prognostics, as described herein. Such diagnostics methods may used to detect the presence or absence of synovial sarcoma and the risk of having the disease. The method for diagnosis and/or prognosis of synovial sarcoma of the present invention comprises immunologically detecting or determining the FZD10 protein derived from the disease in a sample using an antibody or a fragment thereof according to the present invention. Specifically, a method for diagnosis or prognosis of synovial sarcoma or of a predisposition to develop the disease in a subject according to the present invention comprises:
(a) contacting a sample from the subject with an antibody against FZD10 protein or a fragment thereof;
(b) detecting the FZD10 protein in the sample; and
(c) judging whether or not the subject suffers from or is at risk of developing the disease based on the relative abundance of the FZD10 protein compared to a control.

The method for diagnosis and/or prognosis of the present invention can be performed based on any procedures, as long as it is an assay using an antibody, i.e., an immunological assay. Thereby one can detect the FZD10 protein using the antibody or a fragment thereof of the present invention as the antibody used in the assay. For example, the FZD10 protein can be detected by using an immunohistochemical staining, immunoassay such as enzyme immunoassays (ELISA and EIA), immunofluorescent assay, radioimmunoassay (RIA), or Western blotting.

A sample to be tested in the method for diagnosis and/or prognosis of synovial sarcoma of the present invention is not specifically limited, as long as it is a biological sample that may contain the FZD10 protein derived from the synovial sarcoma. Examples of the sample include extract of a cell or organ, and tissue sections, as well as blood, sera, plasma, lymphocyte cultivated supernatant, urine, spinal fluid, saliva, sweat, and ascites. The abundance of the FZD10 protein as determined in samples such as tumor tissue, tumor biopsy, and metastasis tissue by using the antibody or a fragment thereof of the present invention is specifically useful as an index of synovial sarcoma.

For example, antibodies and fragments thereof according to the present invention, such as those described above in Section 5.1., may be used to quantitatively or qualitatively detect the FZD10 protein. The antibodies (or fragment thereof) of the present invention may, additionally, be employed histologically, as in immunofluorescence or immunoelectron microscopy, for *in situ* detection of FZD10 protein. *In situ* detection may be accomplished by removing a histological sample from a subject, such as paraffin-embedded sections of tissues (such as surgical specimens) and applying thereto a labeled antibody of the present invention. The antibody (or fragment thereof) is preferably applied by overlaying a sample with the labeled antibody (or fragment thereof). Using the present invention, those skilled in the art will readily perceive that any of a wide variety of histological methods (such as staining procedures) can be modified in order to achieve such *in situ* detection.

Immunoassays for FZD10 protein will typically comprise incubating a sample from a subject to be examined, such as a biological fluid, a tissue extract, freshly harvested cells, or lysates of cells that have been incubated in cell culture, in the presence of a detectably labeled antibody of the present invention, and detecting the bound antibody by any of a number of techniques well-known in the art.

The sample may be brought into contact with and immobilized onto a solid phase support or carrier such as nitrocellulose, or another solid support which is capable of immobilizing cells, cell particles, or soluble proteins. The support may then be washed with suitable buffers followed by treatment with the detectably labeled antibody against FZD10. The solid phase support may then be washed with the buffer a second time to remove unbound antibody. The amount of bound label on the solid support may then be detected by conventional means.

The term "solid phase support or carrier" means any support capable of binding an antigen or an antibody. Those skilled in the art will know many suitable carriers for binding antibodies or antigens, or will be able to ascertain the same by use of routine experimentation.

The binding activity of a given lot of anti-FZD10 antibody may be determined according to well-known methods. Those skilled in the art will be able to determine operative and optimal assay conditions for each determination by employing routine experimentation.

To detect a reaction between the antibody (or its fragment) of the present invention and the FZD10 protein derived from an synovial sarcoma affected site in a sample easily, the reaction can be directly detected by labeling the antibody of the present invention or indirectly detected by using a labeled secondary antibody. The latter indirect detection procedure, such as a sandwich assay or competitive assay of ELISA, is preferably used in the method of the present invention for better sensitivity.

Examples of labels for use herein are as follows. Peroxidases (PODs), alkaline phosphatases, β-galactosidase, urease, catalase, glucose oxidase, lactate dehydrogenase, amylases, and biotin-avidin complexes can be used in an enzyme immunoassay. Fluorescein isothiocyanate (FITC), tetramethylrhodamine isothiocyanate (TRITC), substituted rhodamine isothiocyanate, and dichlorotriazine isothiocyanate can be used in an immunofluorescent assay. Tritium, ¹²⁵I, and ¹³¹I can be used in a radioimmunoassay. NADH- FMNH₂-luciferase assay, luminol-hydrogen peroxide-POD system, acridinium esters, and dioxetane compounds can be used in an immunoluminescent assay.

The label can be attached to the antibody according to a conventional procedure. For example, the label can be attached to the antibody by a glutaraldehyde method, maleimide method, pyridyl disulfide method, or periodate method in the enzyme immunoassay, and by a chloramine T method or Bolton-Hunter method in the radioimmunoassay.

The assay can be performed according to a known procedure (Ausubel, F.M. et al. Eds., Short Protocols in Molecular Biology, Chapter 11 "Immunology" John Wiley & Sons, Inc. 1995).

For example, when the antibody of the present invention is directly labeled with the label described above, the sample is brought into contact with the labeled antibody to thereby form a complex between the FZD10 protein and the antibody. Then, unbound labeled antibody is separated, and the level of the FZD10 protein in the sample can be determined based on the amount of the bound labeled antibody or that of the unbound labeled antibody.

When a labeled secondary antibody is used, the antibody of the present invention is allowed to react with the sample in a primary reaction, and the resulting complex is allowed to react with the labeled secondary antibody in a secondary reaction. The primary reaction and the secondary reaction can be performed in reverse order, concurrently with some interval of time therebetween. The primary and secondary reactions yield a complex of [FZD10 protein]-[the antibody of the invention]-[the labeled secondary antibody] or a complex of [the antibody of the invention] - [FZD10 protein]-[the labeled secondary antibody]. Unbound labeled secondary antibody is then separated, and the level of the FZD10 protein in the sample can be determined based on the abundance of the bound labeled secondary antibody or that of the unbound labeled secondary antibody.

One of preferred embodiments of the present invention will be illustrated below. Initially, the antibody of the present invention as a primary antibody is brought into contact with a sample such as a tissue section. Unspecific binding of the primary antibody is then blocked using a known blocking reagent. Next, the sample is brought into contact with a labeled secondary antibody that reacts with the primary antibody (the antibody of the present invention) at a site different from that of a FZD10 protein. Signals from the label are then detected.

The "secondary antibody that reacts with the primary antibody at a site different from that of a FZD10 protein" for use herein is not specifically limited, as long as it is an antibody that recognizes a site other than the binding site between the primary antibody and the FZD10 protein. The secondary antibody just mentioned above can be any of polyclonal antibodies, antisera, and monoclonal antibodies, as well as fragments of these antibodies such as Fab fragment, F (ab')₂ fragment, and Fab' fragment. The secondary antibody can be a mixture of two or more types of antibodies.

Thus, the FZD10 protein abundance in the sample from a subject is determined, and whether or not the subject suffers from or is at the risk of developing synovial sarcoma can be judged based on the relative abundance of the FZD10 protein, where necessary as compared with a control, including the protein abundance in a normal sample or a sample of a tissue in which the FZD10 protein is not expressed. As apparent to those skilled in the art, the FZD10 protein abundance varies depending on the conditions, sex, age, and other factors in each subject. Accordingly, the presence of the disease or the risk thereof can be determined preferably by comparing the FZD10 protein abundance in the sample with that in a normal sample or a sample of a tissue in which the FZD10 protein is not expressed, and determining the difference between the two samples. To perform a prognosis, it is also effective to compare the FZD10 protein abundance in the sample with that in a sample collected when the subject has suffered from a primary tumor.

According to another embodiment, the antibody of the present invention is labeled with a radioisotope, and the labeled antibody is parenterally administered to a subject. Thus, the localization of a primary tumor and the related metastasized tumor of synovial sarcoma can be rapidly found in a non-invasive manner. Such a diagnosis method is known as tumor imaging, and one skilled in the art can easily understand the procedures thereof. The labeled antibody can be administered to the subject systemically or locally, preferably through a parenteral route such as intravenous injection, intramuscular injection, intraperitoneal injection, or subcutaneous injection.

### 5.3.1. KITS

The antibodies according to the present invention specifically react with a FZD10 protein as mentioned above and can thereby be used in kits for diagnosis and/or prognosis of synovial sarcoma.

The kit for diagnosis and/or prognosis of the present invention comprises an antibody of the present invention described in Section 5.1. By detecting the FZD10 protein in a sample from a subject who is suspected to suffer from synovial sarcoma with the use of the kit for diagnosis and/or prognosis of the present invention, whether or not the subject suffers from the FZD10-associated disease can be rapidly and easily ascertained. Kits for diagnosis and/or prognosis of diseases using such immunological reactions have been widely known, and one skilled in the art can easily select appropriate components other than the antibody. The kits for diagnosis and/or prognosis of the present invention can be used in any means, as long as it is a means for immunoassay.

### 6. EXAMPLES

The present invention will be further illustrated by the following non-limiting examples:

### Example 1

### Recombinant protein and polyclonal antibody

The recombinant protein of the N-terminal extracellular domain of FZD10 (FZD10-ECD; residues 1-225 of the amino acid sequence shown in SEQ ID NO: 1) fused with His tag was produced in *E*. *coli* using the pET28 expression system (Novagen, Madison, WI). Briefly, expression of the protein was induced by 0.5mM isopropyl β-D-thiogalactopyranoside (IPTG) by incubation at 25 °C for 3 h and then purified with Ni-NTA resin (QIAGEN, Valencia, CA) according to the manufacturer's instructions. Rabbits were immunized with the purified recombinant protein (Medical & Biological Laboratories, Nagoya, Japan), and then the high-titer antiserum was purified using Affi-Gel 15 support (Bio-Rad, Hercules, CA), which was coupled with FZD10-ECD recombinant protein in a coupling solution (20 mM HEPES, 150 mM NaCl, pH 8.0) . Antibody bound to the gel was eluted from the column in 0.1 M glycine (pH 2.5) and immediately neutralized in 1 M Tris (pH 8.5) . The quality and specificity of the affinity-purified polyclonal antibody (hereinafter referred to "TT641 pAb") was verified by SDS-polyacrylamide gel electrophoresis (PAGE) and Western blotting.

### Example 2

### Expression of FZD10

### (1) Preparation of cell lines and tissue specimens

SS cell lines (HS-SY-2, YaFuSS and SYO-1), colon cancer cell lines (SW480, LoVo, DLD1, HT29, HCT116, SNU-C4 and SNU-C5), a cervical adenocarcinoma cell line (HeLa), a fibrosarcoma cell line (HT1080), and COS7 were grown in monolayers in appropriate media supplemented with 10% fetal bovine serum and 1% antibiotic/ antimycotic solution (Sigma, St Louis, MO) and maintained at 37 °C in air containing 5% CO₂. Tumor samples were snap-frozen in liquid nitrogen immediately after resection and stored at -80 °C until preparation of RNA. Surgical specimens were also fixed in 10% formalin and routinely processed for paraffin embedding. The paraffin block was thin-sectioned serially to 5 µm thickness and stained with HE (hematoxylin-eosin) for pathological evaluation. All samples were approved for our analysis by the ethical committee of the Faculty of Medicine, Kyoto University.

### (2) Northern-blot analysis

Total RNAs were extracted from cell lines and from frozen surgical specimens prepared in (1) above using TRIzol reagent (Invitrogen, Carlsbad, CA) according to the manufactures' s instructions. After treatment with DNase I (Nippon Gene, Osaka, Japan), mRNA was isolated with Micro-FastTrack (Invitrogen) following the manufacturer's instructions. A 1-µg aliquot of each mRNA, along with human normal tissue poly A (+) RNA isolated from the heart, brain, lung, liver, kidney, bone marrow, pancreas and placenta (Clontech, Palo Alto, CA) was separated on a 1% denaturing agarose gel and transferred to a nylon membrane. Hybridization with a random-primer α³²P-dCTP labeled FZD10 cDNA probe was carried out according to the instructions for the Megaprime DNA labeling system (Amersham Bioscience). Prehybridization, hybridization, and washing were performed according to the supplier's recommendations. The blots were autoradiographed with intensifying screens at -80 °C for three days.

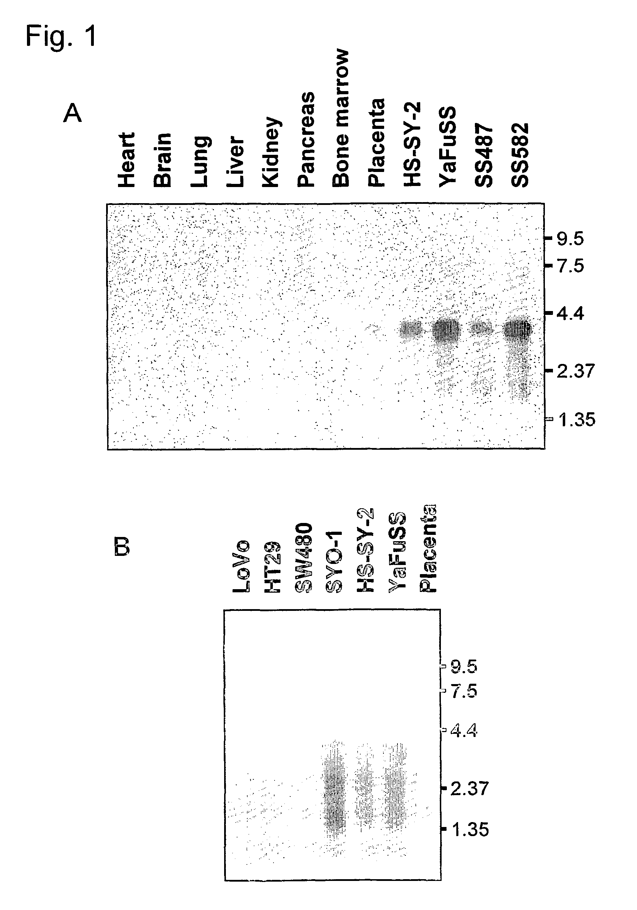

Northern blot analysis revealed that the highest level of FZD10 was expressed in placenta among normal human adult tissues (Fig. 1A), which was consistent with a previous report (Koike, J. et al., Biochem Biophys Res Commun. 262: 39-43., 1999) . As compared to the level of transcripts in placenta, FZD10 gene was expressed in SS cell lines (HS-SY-2 and YaFuSS) and surgical SS specimens (SS487 and SS582) at much higher levels. These findings indicate that transcription of FZD10 increased in SS tumor cells.

### EXAMPLE 3

### Specific recognition of extracellular domain of FZD10 by TT641 pAb

### (1) Western blotting

The present inventors generated the polyclonal antibody that recognized the N-terminal extracellular domain of FZD10 (FZD10-ECD) (see Example 1). To investigate the specificity of the affinity-purified FZD10-ECD antibody (TT641 pAb), Western blotting analyses were performed as following:

For complete solubilization of whole-cell proteins, adherent cells were collected in Laemmli sample buffer (BioRad), lysed with sonication, and boiled for 5 min. Each sample was loaded onto a 10% SDS-PAGE gel, blotted onto a nitrocellulose membrane (Amersham Bioscience) and incubated at 4 °C overnight at 0.1 µg/ml of TT641 pAb. Following 1h of incubation with anti-rabbit HRP-conjugated immunoglobulin (Amersham Bioscience), signals were visualized using enhanced chemiluminescent reagent (ECL) (Amersham Bioscience). As a loading control for proteins, β-actin was detected by monoclonal anti-β-actin AC-15 (Sigma).

As a result, a single 68-kDa band of the predicted size compatible to FZD10 was clearly observed in FZD10-expressing cell lines (Fig. 2A) . However, since the sizes of the other FZD gene family proteins are similar to that of FZD10 protein, we further examined whether the single band recognized by TT641 pAb was specific to the FZD10 protein by semi-quantitative RT-PCR as following.

### (2) Semi-quantitative RT-PCR

A 3-µg aliquot of total RNA prepared from each cell line was reverse-transcribed for single-stranded cDNAs using oligo (dT) 12-18 primer and Superscript II (Invitrogen). Semi-quantitative RT-PCR was carried out with the primers specific to each member of the FZD family or with a β2-microglobulin (β32MG) -specific primer as an internal control. The primer sequences are listed in Table 1.

**Table 1 Primer sequences FZD gene family**

| | Primer F | Primer R |
|---|---|---|
| FZD1 | 5'-CTCGAGGTTTCCTCACTAGACAA-3' (SEQ ID NO: 3) | 5'-AATGGTTAAACCGCCCTAAATAA-3' (SEQ ID NO: 4) |
| FZD2 | 5'-TCCACCTTCTTCACTGTCACC-3' (SEQ ID NO: 5) | 5'-TAAAATACGGAGTCTGTAGGGGC-3' (SEQ ID NO: 6) |
| FZD3 | 5'-ATTGAATAGGCCTGATCATCTGA-3' (SEQ ID NO: 7) | 5'-ATAGGAGCGTAGAGTGCACAAAG-3' (SEQ ID NO: 8) |
| FZD4 | 5'-ATGACTTACAGATCCCCCGAC-3' (SEQ ID NO: 9) | 5'-ACAGAGCAGGGGAAGTCACAT-3' (SEQ ID NO: 10) |
| FZD5 | 5'-CTGCGCTTCTTCCTATGCACTA-3' (SEQ ID NO: 11) | 5'-TTGTTGTAGAGCGGGTGTGACT-3' (SEQ ID NO: 12) |
| FZD6 | 5'-CGCTACTTTGTACTCTTGCCACT-3' (SEQ ID NO: 13) | 5'-ACATGGGATATGGTACTGACGAC-3' (SEQ ID NO: 14) |
| FZD7 | 5'-GCGAGGCGCTCATGAACAAGT-3' (SEQ ID NO: 15) | 5'-CACGGCCACCATGAAGTAGCA-3' (SEQ ID NO:16) |
| FZD8 | 5'-GACACTTGATGGGCTGAGGTTC-3' (SEQ ID D10: 17) | 5'-TAAGTCAGGGGTGGGAGTTTAC-3' (SEQ ID NO: 18) |
| FZD9 | 5'-CTGCACGCTGGTCTTCCTACT-3' (SEQ ID NO: 19) | 5'-CCGATCTTGACCATGAGCTTC-3' (SEQ ID NO: 20) |
| FZD10 | 5'-TCAGAAACCCTTCAGTGCTACAT-3' (SEQ ID NO:21) | 5'-ATACACACGCAGAAACCACTCTT-3' (SEQ ID NO:22) |

| | | |
|---|---|---|
| Primer F or R; forward or reverse primer sequences, respectively | | |

The results are shown in Fig. 2B. The expression pattern found in Western blotting (Fig. 2A) was compared to the levels of transcripts in 10 members of the FZD family using various cell lines (Fig. 2B). As shown in Figs. 2A and 2B, the expression pattern detected with Western blotting using TT641 pAb was quite similar to those of the FZD10 transcripts revealed by semi-quantitative RT-PCR among 10 members of the FZD family, especially in the HeLa and LoVo cell lines. This finding indicates that TT641 pAb recognized the specific epitope of FZD10 but not other members of the FZD gene family.

### EXAMPLE 4

### Localization of FZD10 in cells

### (1) Immunocytochemistry

To examine the subcellular localization of FZD10 protein, we performed immunocytochemical analysis. We initially established COS7-FZD10 cells (S5, S9, S10, S3 and S11) that stably over-expressed FZD10 by transfecting an expression construct of pCAGGS-FZD10-MyC-His into COS7 cells (Fig. 3A).

Firstly, the entire coding sequence of FZD10 cDNA was amplified by RT-PCR using KOD-Plus DNA polymerase (TOYOBO, Osaka, Japan) and inserted into the unique EcoRI site of the pCAGGS mammalian expression vector, which carries a CAG (cytomegalovirus immediate-early enhancer chicken β-actin hybrid) promoter (Niwa, H. et al, Gene. 108: 193-9., 1991) and a gene conferring neomycin resistance. Myc and His epitope tags were placed at the C terminus of the expression vector (pCAGGS-FZD10-MyC-His). Constructs were confirmed by DNA sequencing with forward and reverse primers; forward, 5'-GTCCCCTTCTCCATCTCCAG-3' (SEQ ID NO: 23); reverse, 5`-TATTTGTGAGCCAGGGCATT-3'(SEQ ID NO: 24).

Then, COS7 cells were seeded at 5 x 10⁴ cells per six-well plate. After 24 h, cells were transfected with 2 µg of pCAGGS-FZD10-MyC-His mixture pre-incubated for 15 min with 6 µl of FuGene6 transfection reagent (Roche, Basel, Switzerland). Following a three-week culture period in selective medium containing 0.4 mg/ml of Geneticin (Invitrogen), stable transfectants were established.

COS7-derived stable transfectants expressing FZD10 were fixed with 4% paraformaldehyde in PBS and then covered with blocking solution (3% BSA) for 1 h at room temperature. To minimize cell lysis, permealization with detergents was not performed. The cells were then incubated with mouse anti-c-myc antibody (9E10, diluted 1:1000) and with the TT641 pAb (2 µg/ml) in blocking solution at 4 °C overnight. Primary antibodies were stained with goat anti-rabbit secondary fluorescent antibodies (Alexa Flour 488; diluted 1:500, Molecular Probes) and horse anti-mouse secondary antibodies conjugated with Texas Red (diluted 1:1000, Vector Laboratories, Burlingame, CA) for 1 h at room temperature, stained with DAPI (4',6-diamidino-2-phenylindole) and visualized with an ECLIPSE E 600 microscope (Nikon, Tokyo, Japan). To detect the endogenous expression patterns of FZD10, SS cell lines (HS-SY-2 and YaFuSS) were also fluorescence immunostained with 2 µg/ml of TT641 pAb in the same manner.

When COS7-FZD10 cells were counterstained with Texas Red-conjugated anti-myc antibody, the red signal coincided with the green one of the TT641 pAb (Fig. 3B), supporting the specific binding of the TT641 pAb to FZD10. Furthermore, the immunocytochemical analysis using TT641 pAb revealed that endogenous expression patterns observed in SS cell lines (HS-SY-2 and YaFuSS) were similar to those in stable transfectans (Fig. 3C). The reason why FZD10 stained in a dotted pattern in the cytoplasm remains unclear, although the predicted FZD10 protein is known to be a seven-pass transmembrane receptor (Koike, J. et al., Biochem Biophys Res Commun. 262: 39-43., 1999). Presumably, the mature cell-surface antigen appeared in relatively low concentrations and abundant unprocessed antigens in the cytoplasm may be detected in immunocytochemistry.

### (2) Flow cytometric analysis

To address the question as to the subcellular localization raised in the immunocytochemistry section, flow cytometric analysis was performed.

5 x 10⁶ cells were collected by trypsinization and incubated with 1.5 µg of TT641 pAb and non-immunized rabbit IgG (DAKO, Kyoto, Japan) at 4 °C for 30 min. After washing 3 times with PBS, 2 µg of fluorescent anti-rabbit IgG (Alexa Fluor 488, Molecular Probe) was added to the cell suspension and incubated at 4 °C for 30 min. Immediately after washing three times with PBS, cells were analyzed by a FACScan (Becton Dickinson, San Jose, CA).

Three SS cell lines, YaFuSS, HS-SY-2, and SYO-1, were specifically labeled with the TT641 pAb (Fig. 4), whereas no fluorescence signals were detected in SW480, HT29, or LoVo cell lines. These observations were correlated with the expression levels of FZD10 observed in Northern blots (see, Example 1, Fig. 1B) . Taken together, these findings indicate that the TT641 pAb specifically recognizes the cell-surface antigen of FZD10, but not any other FZD members (Figs. 2A and 2B), under both native and denaturing conditions.

### Example 5

### Epitope mapping of TT641 pAb

To characterize the specificity of TT641 pAb, we initially performed epitope mapping using SPOTs system as following:

A series of 10-residue linear peptides overlapping by one amino acid and covering the entire sequence of FZD10-ECD (residues 1-225 of the amino acid sequence shown in SEQ ID NO: 1) was synthesized and covalently bound to a cellulose membrane by the SPOT synthesis technique (SPOTs; Sigma Genosys). According to the manufacturer's recommendations, the membrane containing 216 peptide spots was preincubated for 8 h at room temperature with a blocking buffer (Sigma) and hybridized with TT641 pAb in the blocking buffer at 4 °C overnight. The membrane was washed with 0.05% Tween 20/TBS (50 mM Tris, 137 mM NaCl and 2.7 mM KCl, pH 8.0), followed by 2 h of incubation of anti-rabbit immunoglobulin conjugated with horseradish peroxidase (HRP) (Amersham Bioscience) in the blocking buffer at room temperature. After three washes with 0.05% Tween 20/TBS, the spots were visualized with signal development solution (Sigma) containing 3-amino-9-ethylcarbazole.

As a result, TT641 pAb recognized 6 different epitopes of FZD10-ECD to a different degree (Fig. 5). Among them, TT641 pAb showed the strongest reactivity to the epitope ranging from 214-225 residues, which was thought to represent a critical sequence for specific binding of TT641 pAb to FZD10-ECD.

### Example 6

### Expression pattern of FZD10 protein

### (1) Immunohistochemical staining

To investigate whether TT641 pAb could specifically recognize FZD10 protein in tissue sections, we initially performed immunohistochemical analyses in normal adult human tissues and SS surgical specimens using the TT641 pAb.

Each serial section of the paraffin-embedded specimens was mounted on a silanized slide, deparaffinized in xylene and rehydrated in phosphate buffered saline (PBS). The sections were then processed for antigen retrieval by microwave treatment. After quenching endogenous peroxidase activity with 3% hydrogen peroxide, non-specific binding of primary antibodies was blocked with a blocking reagent (DAKO). The slides were then incubated at 4 °C overnight with TT641 pAb at 5 µg/ml. Subsequently, rabbit ENVISION Polymer Reagent (DAKO) was added as a secondary antibody for 60-mins reaction at room temperature. Finally the immunoreaction was visualized with peroxidase substrate 3, 3'-diaminobenzidine tetrahydrochloride (DAKO). The sections were counterstained with hematoxylin, dehydrated in graded alcohols, cleared in xylene, and coverslipped. Negative controls were run in parallel with replacement of the specific antibody with non-immune normal rabbit IgG (DAKO). Paraffin-embedded slides of human adult normal tissues were purchased from DAKO and BioChain (Hayward, CA), and immunostained in the same manner as mentioned above.

In contrast to positive staining for FZD10 in placenta (Fig. 6A), no expression of FZD10 was detected by immunostaining with TT641 pAb in five different tissue sections of normal brain (Fig. 6B), heart (Fig. 6C), lung (Fig. 6D) or liver (Fig. 6E), as expected from the Northern blots (see, Example 2, Fig. 1A). In normal kidney, however, positive staining was observed in the proximal and distal tubules and collecting tubes (Fig. 6F), although the degree of staining intensity varied between individuals (Fig. 6G). In normal stomach tissues, strong immunoreactivity was observed in the upper portion of gastric glands, but the staining intensity was much weaker in cells located at the bottom of the glands (Figs. 6H and 6I). In normal colon tissues as well, epithelial cells showed faint immunoreactivity of FZD10 at the bottom of the crypts, but strong staining intensity was detected at the surface of the villi (Fig. 6J). In contrast, strong expression of FZD10 was found in a cytoplasmic pattern in SS tumor cells of the biphasic SS specimen (Figs. 6K and 6L). It is noteworthy that staining intensity was especially strong in epithelial tumor cells, whereas non-epithelial spindle tumor cells showed faint immunoreactivity. These data suggested that expression levels of FZD10 protein was also absent or low in normal vital organs, compared to the increased expression in SS tissues. In addition, colon cancer cells in the primary and metastatic lesions were also specifically immunostained with TT641 pAb, but not detectable signals in the surrounding stromal and liver tissues (Figs. 6M, 6N and 6O).

### Example 7

### TT641 pAb mediates ADCC against FZD10-expressing SS cells

To further examine whether the TT641 pAb induces antibody-dependent cell-mediated cytotoxicity (ADCC) against SS cells, we measured LDH release from SS cells upon cell lysis.

Cytotoxicity was assayed by quantitative measurement of lactate dehydrogenase (LDH), a stable cytosolic enzyme that is released upon cell lysis, using CytoTox96 Non-radioactive Cytotoxicity Assay (Promega, Madison, WI). For the preparation of fresh effector cells, peripheral blood mononuclear cells (PBMCs) were isolated from heparinized peripheral blood of a healthy donor by Ficoll-Paque (Amersham Bioscience) density gradient centrifugation. Following the manufacturer's instructions, effector cells (E) and target cells (T) (5 x 10³/well) were co-incubated at various E:T ratios together with TT641 pAb or non-immunized rabbit IgG in 100 µl of phenol red-free RPMI 1640 supplemented with 5% FBS in a 96-well round-bottom plate in quadruplicate for 6 h at 37°C. Released LDH in the culture supernatant (50 µl) was measured by a colorimeric assay, which results in the conversion of a tetrazolium salt into a red formazan product. Absorbance data at 490 nm were collected with a standard 96-well plate reader. After the data were corrected for background signals, the percentage of specific cytotoxicity was calculated according to the formula: % cytotoxicity = 100 x (experimental LDH release - effector spontaneous LDH release - target spontaneous LDH release) / (target maximal LDH release - target spontaneous LDH release). Controls included the incubation of either target or effector cells with TT641 pAb.

As shown in Fig. 7A, when target and effector cells were co-incubated with 7 µg/ml (0.7 µg/well) of TT641 pAb at an E:T ratio of 25:1, there were no cytotoxic effects in the target cell line (SYO-1) with TT641 pAb alone (T + Ab), and no evidence of cytotoxic interaction between the TT641 pAb and the human effector cells (E +Ab) or between the target cells and the human effector cells (T + E). On the other hand, cytotoxic effects were observed when the target cells were incubated with both the antibody and human effector cells (T+E+Ab). Even when the target cells were incubated with different concentrations of TT641 pAb in different E:T ratios, cytotoxicity was induced only when both the antibody and human effector cells were added at the same time.

As shown in Fig. 7B, 1 µg of the TT641 pAb induced 78% of cell-mediated cytotoxicity against FZD10-overexpressing cells at an E:T ratio of 25:1. This cytotoxic effect was positively correlated with the E:T ratios and the amount of the antibody added. There was no significant ADCC induced by control antibody against the target cells. These results suggested the possibility that TT641 pAb against FZD10 could exhibit a growth inhibitory effect for FZD10-overexpressing tumors through ADCC.

### EXAMPLE 8

### Growth inhibitory effect of TT641 pAb on SS xenografts

In this Example, mice were inoculated subcutaneously with SYO-1 cells to examine the growth inhibitory effect of TT641 pAb on SS xenografts.

Animal work was performed in the animal facility in accordance with institutional guidelines. Female 6-week-old athymic mice (BALB/cA Jcl-*nu*) were used. Mice were acclimated and housed in sterile cages in groups of 3 under laminar flow hoods in a temperature-controlled room with a 12-hour light/12-hour dark schedule, and fed autoclaved chow and water *ad libitum.*

For the cell implantations, SYO-1 cells, grown in monolayers, were trypsinized and resuspended in serum-free medium. The final concentration was adjusted to 5 x 10⁷ cells/ml and the cell suspension was placed on ice. After the site was cleaned with ethanol, 0.1 ml (5 x 10⁶ cells) of the suspension was subcutaneously injected into the flanks of nude mice. Tumors were measured with a dial-caliper, and volumes were determined using the formula: 0.5 x (larger diameter) x (smaller diameter)². When the primary tumors were 40-75 mm³ in size, animals were randomly divided into two groups. One group (n = 16) received intratumoral injection of 10 µg of the TT641 pAb as a suspension in 75 µl of PBS for 5 consecutive days (Days 0-4). As a control, the other group (n = 15) received non-immunized rabbit IgG (DAKO). Tumor growth was assessed by calculation of a growth ratio based on tumor volume at the indicated day to that calculated at the initial day of treatment.

As shown in Fig. 8, the growth of SS xenografts was attenuated by treatment with TT641 pAb, as compared with treatment with non-immunized rabbit IgG. At day 6 after the initiation of antibody injection, the growth rate of SS xenografts in mice treated with TT641 pAb was significantly lower than that observed in negative controls (*P* = 1.71 x 10⁻⁵; Student's t-test).

To elucidate the reason of growth attenuation by treatment with TT641, The present inventors performed the TUNEL analysis.

Mice were sacrificed at the indicated time, and the tumors were collected and fixed with 10% formaldehyde. For the *in situ* terminal transferase-mediated dUTP nick end-labeling (TUNEL) assay, one of the serial sections of paraffin-embedded specimens was stained using ApopTag Apoptosis Detection Kit (Intergen) according to the manufacturer's instructions. In addition, to assess cell proliferation ability, immunohistochemical staining with anti-Ki-67 mouse monoclonal antibody (MIB-1, DAKO) was carried out in the same manner as mentioned in the immunohistochemical staining section.

The specimen of tumor tissues treated with TT641 pAb showed clusters of apoptotic cells (Figs. 9F and 9L), which were negative for staining for a marker of cell proliferation, Ki-67 (Figs.9E and 9K), whereas apoptotic cells were sparse in tumor specimens of negative controls (Figs. 9C and 9I). Since the apoptotic cells in the tumor tissue were surrounded by many viable tumor cells, which were positive for Ki-67 staining (Figs. 9E and 9K), the growth inhibitory effect of TT641 pAb on SS xenografts was thought to be insufficient to regress the tumors drastically.

### EXAMPLE 9

### Expression of mouse FZD10 in normal mouse tissues

Since it was revealed that an amino-acid identity between human and mouse FZD10 was approximately 93%, we investigated whether TT641 pAb could also cross-react with mouse FZD10 protein using normal mouse tissues by Northern blot and immunohistochemical analyses. Although Northern-blot analysis revealed that no band was detected in normal mouse heart, brain, spleen, lung, liver skeletal muscle, kidney or testis tissues (Fig. 10A), immunohistochemical analysis showed that positive staining was observed in mouse kidney (Fig. 10B), and placenta (Fig. 10C), as well as in normal human tissues, and weak immunoreactivity was observed in normal mouse lung (Fig. 10D), but not observed in brain (Fig. 10E).

### EXAMPLE 10

### Generation of monoclonal antibodies (mAbs)

Monoclonal anti-FZD10 antibodies (mAbs 1F2, 1F4, 5F2, 5H4 and 6C9) were obtained in the following:

A purified FZD10-ECD (residues 1-225 of the amino acid sequence shown in SEQ IS NO: 1) recombinant protein (Medical & Biological Laboratories, Nagoya, Japan) was used to immunize three 6-week BALB/c mice. 100 mg of the antigen (FZD10-ECD recombinant protein) emulsified in Freund's complete adjuvant was injected into the both foot pads of each mouse, followed by three times intraperitoneal injections at 3 days intervals. Two days after one booster inoculation of 100 mg of in 150ml of Freund's complete adjuvant, cell fusion and cloning by limiting dilution were performed as follows. Total of six lymph nodes from selected mouse were fused with P3-U1 myeloma cells and hybridomas selected with HAT (15%FCS/RPMI/HAT/ BM-condimedH1) medium. After about 2-weeks from the fusion, the supernatants of the cells were screened by ELISA assay using recombinant FZD10-ECD as antigen. 42 positive hybridomas (OD450 in ELISA > 0.1) were cloned twice by limiting dilution, and further selected by flow cytometry analysis using SS cell line, YaFuSS. Eventually, five single clones were selected as monoclonal antibody-producing hybridoma. Heavy chain isotype of each clone was identified by ELISA and monoclonal antibody concentration was determined by immunodiffusion. To produce large quantities of mAbs, 5 X 105 cloned hybridoma cells were administered into ascites fluid in BALB/c mice. After 10 days to 2-weeks after administration, ascites fluid were collected and pooled.

### EXAMPLE 11

### Specificity of monoclonal anti-FZD10 antibodies (mAbs)

### (1) Specificity to FZD10 protein

We first examined the specificity of the mAbs obtained in Example 10 by flow cytometry analysis using SS cell lines (SYO-1 and YaFuSS) which showed high expression of FZD10 transcript, and colon-cancer cell lines (SW480 and HT29) which showed hardly detectable expression. All of these mAbs could specifically recognize the FZD10 protein as surface antigen in SYO-1 or YaFuSS, whereas no fluorescent signals were detected in SW480 or HT29 (Figs. 11A(1F2), 11B(1F4), 11C(5F2), 11D(5H4), 11E(6C9)). These results are consistent with those obtained by using TT641 pAb as described in Example 4. Taken together, these results indicate that the anti-FZD10 mAbs also specifically recognize the extracellular domain of FZD10 under native condition.

### (2) Epitope mapping

To further characterize the specificity of each mAb, we performed epitope mapping in the same manner as in Example 5.

A series of 10-residue linear synthetic peptides overlapping by one amino acid and covering the entire FZD10-ECD was covalently bound to a cellulose membrane (SPOTs; Sigma Genosys, Woodlands, Texas) as described in Example 5. The membrane containing 216 peptide spots was hybridized with anti-FZD10 mAbs (1F2, 5F2, 5H4 and 6C9) at 4 °C overnight. After incubation with anti-rabbit HRP-conjugated IgG (Amersham Bioscience, Piscataway, New Jersey), the spots were visualized with 3-amino-9-ethylcarbazole.

As a result, mAbs 1F2, 5F2, 5H4 and 6C9 recognized the amino acid residues 157-170 (EPTRGSGLFPPLFR), 157-170 (EPTRGSGLFPPLFR), 161-173 (GSGLFPPLFRPQR), 156-169 (DEPTRGSGLFPPLF) in FZD10-ECD (residues 1-225 of the amino acid sequence shown in SEQ ID NO:1), respectively (Fig. 12).

### EXAMPLE 12

### Inhibition of growth of SS xenografts by 5F2 mAb

*In vivo* experiments were performed in our animal facility in accordance with institutional guidelines as well. A 0.1ml of SYO-1 cell suspension (5 x 10⁶ cells) was injected subcutaneously into the flanks of six-week-old athymic female mice (BALB/cA Jcl-nu) . Tumor volumes were determined using the formula: 0.5 x (larger diameter) x (smaller diameter)². When the xenografts were 40-75 mm³ in size, animals were randomly divided into two groups. One group received intratumoral injection of 50µg of the mAbs, 5F2 (n=5), for 10 consecutive days, respectively. As a control, the other group (n=6) received non-immunized rabbit IgG (DAKO). Tumor growth was assessed by calculating the ratio of tumor volume on the indicated day to the volume calculated on the initiation of treatment.

To confirm the growth inhibition effect of TT641 pAb in SS xenografts, we examined whether the anti-FZD10 mAbs, 5F2 and 1F2 show antitumor effect in SS xenografts. The growth of SS xenografts was attenuated by treatment with mAb 5F2, as compared to treatment with non-immunized rabbit IgG (Fig. 13). At day 10 after initiation of antibody treatment (as indicated by the arrows in Fig. 13), the growth rate of SS xenografts in mice treated with mAbs 5F2 was significantly lower than that observed in negative controls (Fig. 13). These findings indicated that mAb 5F2 is likely to inhibit the tumor growth of SS cells.

## Claims

1. An antibody or antibody fragment against Frizzled homologue 10 (FZD10) protein for use in a method of treating or preventing synovial sarcoma.

2. An *in vitro* method for diagnosis or prognosis of synovial sarcoma, or of a predisposition to developing synovial sarcoma in a subject, comprising:
(a) contacting a sample from the subject with an antibody or antibody fragment against FZD10 protein;
(b) detecting the FZD 10 protein in the sample; and
(c) judging whether or not the subject suffers from or is at risk of developing synovial sarcoma based on the relative abundance of the FZD10 protein compared to a control.

3. The antibody of claim 1 or the method of claim 2, wherein the antibody is polyclonal or monoclonal antibody.

4. The antibody of claim 1 or the method of claim 2, wherein the antibody is raised against a peptide comprising at least residues 43-56, 61-72, 156-169, 157-170, 157-172, 161-173, 174-191, 189-202, 214-225, or 1-225 of an amino acid sequence shown in SEQ ID NO: 1.

5. A pharmaceutical composition for use in treating or preventing synovial sarcoma, comprising an antibody or an antibody fragment that is raised against a peptide comprising at least residues 43-56, 61-72, 156-169, 157-170, 157-172, 161-173, 174-191, 189-202, 214-225, or 1-225 of an amino acid sequence shown in SEQ ID NO: 1, and a pharmaceutically acceptable carrier or excipient, wherein said antibody or antibody fragment specifically recognise FZD10 and does not recognise other members of the FZD family.

6. A kit for diagnosis or prognosis of synovial sarcoma, comprising an antibody or an antibody fragment that is raised against a peptide comprising at least residues 43-56, 61-72, 156-159, 157-170, 157-172, 161-173, 174-191, 189-202, 214-225, or 1-225 of the amino acid sequence shown in SEQ ID NO: 1, wherein said antibody or antibody fragment specifically recognise FZD10 and does not recognise other members of the FZD family.

7. An antibody or an antibody fragment against Frizzled homologue 10 (FZD10) protein, which is raised against a peptide comprising at least residues 43-56, 61-72, 156-169, 157-170, 157-172, 161-173, 174-191, 189-202, 214-225, or 1-225 of an amino acid sequence shown in SEQ ID NO: 1, wherein said antibody or antibody fragment specifically recognise FZD10 and does not recognise other members of the FZD family.

8. The antibody or antibody fragment thereof of claim 7, which is raised against a peptide consisting of residues 1-225 of the amino acid sequence shown in SEQ ID NO: 1.

9. The antibody or antibody fragment thereof of claim 7 or 8, which is a polyclonal or monoclonal antibody.

## Patentansprüche

1. Antikörper oder Antikörperfragment gegen Frizzled-Homologon 10 (F2D10)-Protein zur Verwendung in einem Verfahren zur Behandlung oder Prävention von Synovialsarkom.

2. In-vitro-Verfahren zur Diagnose oder Prognose von Synovialsarkom oder einer Prädisposition für die Entwicklung von Synovialsarkom bei einem Subjekt, umfassend:
(a) Inkontaktbringen einer Probe von dem Subjekt mit einem Antikörper oder Antikörperfragment gegen FZD10-Protein;
(b) Detektieren des FZD10-Proteins in der Probe und
(c) Beurteilen, ob das Subjekt an Synovialsarkom leidet oder ein Risiko zur Entwicklung von Synovialsarkom hat oder nicht, auf der Basis der relativen Abundanz des FZD10-Proteins im Vergleich zu einer Kontrolle.

3. Antikörper gemäß Anspruch 1 oder Verfahren gemäß Anspruch 2, wobei der Antikörper ein polyklonaler oder monoklonaler Antikörper ist.

4. Antikörper gemäß Anspruch 1 oder Verfahren gemäß Anspruch 2, wobei der Antikörper gegen ein Peptid gerichtet ist, das wenigstens Reste 43-56, 61-72, 156-169, 157-170, 157-172, 161-173, 174-191, 189-202, 214-225 oder 1-225 einer in SEQ ID NO:1 dargestellten Aminosäuresequenz umfasst.

5. Pharmazeutische Zusammensetzung zur Verwendung bei der Behandlung oder Prävention von Synovialsarkom, umfassend einen Antikörper oder ein Antikörperfragment, der/das gegen ein Peptid gerichtet ist, das wenigstens Reste 43-56, 61-72, 156-169, 157-170, 157-172, 161-173, 174-191, 189-202, 214-225 oder 1-225 einer in SEQ ID NO:1 dargestellten Aminosäuresequenz umfasst, und einen pharmazeutisch verträglichen Träger oder ein pharmazeutisch verträgliches Exzipiens, wobei der Antikörper oder das Antikörperfragment FZD10 spezifisch erkennt und andere Mitglieder der FZD-Familie nicht erkennt.

6. Kit zur Diagnose oder Prognose von Synovialsarkom, umfassend einen Antikörper oder ein Antikörperfragment, der/das gegen ein Peptid gerichtet ist, das wenigstens Reste 43-56, 61-72, 156-159, 157-170, 157-172, 161-173, 174-191, 189-202, 214-225 oder 1-225 der in SEQ ID NO:1 dargestellten Aminosäuresequenz umfasst, wobei der Antikörper oder das Antikörperfragment spezifisch FZD10 erkennt und andere Mitglieder der FZD-Familie nicht erkennt.

7. Antikörper oder Antikörperfragment gegen Frizzled-Homologon 10 (FD210)-Protein, der/das gegen ein Peptid gerichtet ist, das wenigstens Reste 43-56, 61-72, 156-169, 157-170, 157-172, 161-173, 174-191, 189-202, 214-225 oder 1-225 einer in SEQ ID NO:1 dargestellten Aminosäuresequenz umfasst, wobei der Antikörper oder das Antikörperfragment spezifisch FZD10 erkennt und andere Mitglieder der FZD-Familie nicht erkennt.

8. Antikörper oder Antikörperfragment davon gemäß Anspruch 7, das gegen ein Peptid gerichtet ist, welches aus Resten 1-225 der in SEQ ID NO:1 dargestellten Aminosäuresequenz besteht.

9. Antikörper oder Antikörperfragment davon gemäß Anspruch 7 oder 8, der ein polyklonaler oder monoklonaler Antikörper ist.

## Revendications

1. Anticorps ou fragment d'anticorps dirigé contre la protéine FZD10 (Frizzled homologue 10), pour utilisation dans un procédé de traitement ou de prévention d'un synovialosarcome.

2. Procédé *in vitro* de diagnostic ou de pronostic d'un synovialosarcome ou d'une prédisposition au développement d'un synovialosarcome chez un sujet, lequel procédé comporte :
a) mettre un échantillon provenant du sujet en contact avec un anticorps ou fragment d'anticorps dirigé contre la protéine FZD10 ;
b) détecter la protéine FZD10 dans l'échantillon ;
c) et juger si, oui ou non, le sujet souffre d'un synovialosarcome ou présente un risque d'en développer un, d'après l'abondance relative de la protéine FZD10 par rapport à un témoin.

3. Anticorps conforme à la revendication 1 ou procédé conforme à la revendication 2, dans lequel l'anticorps est un anticorps polyclonal ou monoclonal.

4. Anticorps conforme à la revendication 1 ou procédé conforme à la revendication 2, dans lequel l'anticorps est dirigé contre un peptide comprenant au moins les résidus 43-56, 61-72, 156-169, 157-170, 157-172, 161-173, 174-191, 189-202, 214-225 ou 1-225 de la séquence d'acides aminés présentée en tant que Séquence N° 1.

5. Composition pharmaceutique pour utilisation dans le traitement ou la prévention d'un synovialosarcome, comprenant un anticorps ou un fragment d'anticorps qui est dirigé contre un peptide comprenant au moins les résidus 43-56, 61-72, 156-169, 157-170, 157-172, 161-173, 174-191, 189-202, 214-225 ou 1-225 de la séquence d'acides aminés présentée en tant que Séquence N° 1, ainsi qu'un véhicule ou excipient pharmacologiquement admissible, lequel anticorps ou fragment d'anticorps reconnaît spécifiquement la protéine FZD10 et ne reconnaît pas d'autres membres de la famille des protéines FZD.

6. Trousse conçue pour le diagnostic ou le pronostic d'un synovialosarcome, comprenant un anticorps ou un fragment d'anticorps qui est dirigé contre un peptide comprenant au moins les résidus 43-56, 61-72, 156-169, 157-170, 157-172, 161-173, 174-191, 189-202, 214-225 ou 1-225 de la séquence d'acides aminés présentée en tant que Séquence N° 1, lequel anticorps ou fragment d'anticorps reconnaît spécifiquement la protéine FZD10 et ne reconnaît pas d'autres membres de la famille des protéines FZD.

7. Anticorps ou fragment d'anticorps dirigé contre la protéine FZD10 (Frizzled homologue 10), qui est dirigé contre un peptide comprenant au moins les résidus 43-56, 61-72, 156-169, 157-170, 157-172, 161-173, 174-191, 189-202, 214-225 ou 1-225 de la séquence d'acides aminés présentée en tant que Séquence N° 1, lequel anticorps ou fragment d'anticorps reconnaît spécifiquement la protéine FZD10 et ne reconnaît pas d'autres membres de la famille des protéines FZD.

8. Anticorps ou fragment d'anticorps conforme à la revendication 7, qui est dirigé contre un peptide constitué des résidus 1-225 de la séquence d'acides aminés présentée en tant que Séquence N° 1.

9. Anticorps ou fragment d'anticorps conforme à la revendication 7 ou 8, qui est un anticorps polyclonal ou monoclonal.
